# EUROPEAN PATENT APPLICATION

(11) **EP 4 177 238 A1**
(43) Date of publication of application: **10.05.2023**
(21) Application number: 21862121.7
(22) Date of filing: 27.08.2021
(51) Int. Cl.: C07B 59/00, H01L 51/00

(54) **METHOD FOR PREPARING DEUTERATED AROMATIC COMPOUND AND DEUTERATED COMPOSITION**

(30) Priority: 27.08.2020 KR 20200108194
(71) Applicant: Lg Chem, Ltd., Seoul 07336 (KR)
(72) Inventor: JEONG, Dongmin, Daejeon 34122 (KR); CHOI, Dai Seung, Daejeon 34122 (KR); HWANG, Seungyeon, Daejeon 34122 (KR); KIM, Bethy, Daejeon 34122 (KR)
(74) Representative: Hoffmann Eitle
(86) International application number: PCT/KR2021/011510
(87) International publication number: WO 2022/045825

(57) **Abstract**

The present specification relates to a method for preparing a deuterated aromatic compound and a deuterated reaction composition.

## Description

### Technical Field

This application claims priority to and the benefit of Korean Patent Application No. 10-2020-0108194 filed in the Korean Intellectual Property Office on August 27, 2020, the entire contents of which are incorporated herein by reference.

The present specification relates to a method for producing a deuterated aromatic compound and a deuterated reaction composition.

### Background

Compounds including deuterium are used for various purposes. For example, compounds including deuterium can be frequently used not only as labeling compounds for elucidating the mechanism of a chemical reaction or elucidating a material metabolism, but also for drugs, pesticides, organic EL materials and other purposes.

A method of deuterium substitution of an aromatic compound is known in order to improve the lifespan of an organic light emitting device (OLED) material. The principle of such an effect is that while the LUMO energy of C-D bond is lower than that of C-H bond during deuterium substitution, the life characteristics of the OLED material are improved.

When a deuterated reaction was performed on one or more aromatic compounds using an existing heterogeneous catalytic reaction, there was a problem in that by-products due to side reactions continued to be generated. The by-products are caused by a hydrogenation reaction generated by hydrogen gas, and in order to remove the by-products, attempts have also been made to increase the purity through the purification process after the reaction, but it was difficult to obtain high purity because there was no difference in melting point and solubility from existing materials. When the reaction is performed without hydrogen gas in order to alleviate the problem, the reaction needs to be performed at a very high temperature (about 220°C or higher), which can pose a safety problem in the process.

### Brief Description

### Technical Problem

The present specification has been made in an effort to provide a method for producing a deuterated aromatic compound and a deuterated reaction composition.

### Technical Solution

The present specification provides a method for producing a deuterated aromatic compound, the method including: performing a deuterated reaction of an aromatic compound including one or more aromatic rings using a solution including the aromatic compound, heavy water (D₂O) and an organic compound which can be hydrolyzed by the heavy water.

Further, the present specification provides a deuterated reaction composition including an aromatic compound including one or more aromatic rings, heavy water (D₂O) and an organic compound which can be hydrolyzed by the heavy water.

In addition, the present specification provides a deuterated aromatic compound prepared by the above-described method.

Furthermore, the present specification provides an electronic device including the deuterated aromatic compound.

### Advantageous Effects

A production method of a first exemplary embodiment according to the present specification has an advantage in that impurities due to hydrogen gas are not generated.

A production method of a second exemplary embodiment according to the present specification has an advantage in that the deuterium substitution rate is high.

A production method of a third exemplary embodiment according to the present specification has an advantage in that the purity of an obtained compound is high.

A production method of a fourth exemplary embodiment according to the present specification enables a deuterated reaction under a lower pressure.

A production method of a fifth exemplary embodiment according to the present specification enables a deuterated reaction to be performed at a lower temperature.

### Detailed Description

Hereinafter, the present specification will be described in detail.

The present specification provides a method for producing a deuterated aromatic compound, the method including: performing a deuterated reaction of an aromatic compound including one or more aromatic rings using a solution including the aromatic compound, heavy water (D₂O) and an organic compound which can be hydrolyzed by the heavy water.

The method for producing a deuterated aromatic compound of the present specification is characterized in that there is no hydrogen supply step.

In the related art, hydrogen gas was supplied in order to activate a metal catalyst, which is a heterogeneous catalyst added to produce a deuterated aromatic compound. When a deuterated reaction is performed by supplying hydrogen, a hydrogenation reaction is performed by hydrogen gas and thus by-products are generated by a side reaction.

In order to remove the generated by-products, a process of increasing the purity through a purification process after the reaction is required, and even though the purification process as described above is performed, the by-products have no difference in melting point and solubility from a target material, so that it is difficult to produce the deuterated aromatic compound with high purity.

The method for producing a deuterated aromatic compound of the present specification has an advantage in that impurities due to hydrogen gas are not generated because a metal catalyst and hydrogen gas for activating the metal catalyst need not be supplied due to the use of an organic compound which can be hydrolyzed by the heavy water instead of the metal catalyst which is a heterogeneous catalyst.

Meanwhile, when a metal catalyst is used during the deuterated reaction, the metal catalyst reacts with a reactive group of a compound to be deuterated, that is, a halogen group, a hydroxyl group, and the like, so that in a deuterated reaction using a metal catalyst, the compound to be deuterated has no choice but to be limited to a compound having no reactive group capable of reacting with the metal catalyst, or a reactive group which has low reactivity.

Since an organic compound which can be hydrolyzed by heavy water is used instead of a metal catalyst which is a heterogeneous catalyst in the method for producing a deuterated aromatic compound of the present specification, a compound having a reactive group such as a halogen group and a hydroxyl group can also be selected as the compound to be deuterated. Specifically, after a compound, which is an intermediate having a reactive group such as a halogen group and a hydroxyl group, is deuterated, a reaction of substituting the reactive group with an additional aromatic substituent can be performed.

The production method according to the present specification has an advantage in that the deuterium substitution rate is high.

The production method according to the present specification has an advantage in that the purity of an obtained compound is high.

The production method according to the present specification enables a deuterated reaction to be performed under a lower pressure.

The production method according to the present specification enables a deuterated reaction to be performed at a lower temperature.

The method for producing a deuterated aromatic compound of the present specification includes: preparing a solution including an aromatic compound including one or more aromatic rings, heavy water (D₂O) and an organic compound which can be hydrolyzed by the heavy water.

The preparing of the solution including the aromatic compound including one or more aromatic rings, heavy water (D₂O) and the organic compound which can be hydrolyzed by the heavy water can prepare the solution by introducing a solution including an aromatic compound including one or more aromatic rings, heavy water (D₂O) and an organic compound which can be hydrolyzed by the heavy water into a reactor, or individually introducing an aromatic compound including one or more aromatic rings, heavy water (D₂O) and an organic compound which can be hydrolyzed by the heavy water into a reactor.

The organic compound which can be hydrolyzed by the heavy water is not particularly limited as long as the organic compound has a reactive group which can be decomposed by heavy water, and the organic compound can include, for example, at least one compound of the following Chemical Formulae 1 to 4:

[Chemical Formula 1] R1-C(O)OC(O)-R2

[Chemical Formula 2] R3-S(O₂)OS(O₂)-R4

[Chemical Formula 3] R5-C(O)O-R6

[Chemical Formula 4] R7-CONH-R8

wherein in Chemical Formulae 1 to 4, R1 to R8 are the same as or different from each other, and are each independently a monovalent organic group.

In an exemplary embodiment of the present specification, R1 and R2 can be the same substituent.

In an exemplary embodiment of the present specification, R3 and R4 can be the same substituent.

In an exemplary embodiment of the present specification, R5 and R6 can be the same substituent.

In an exemplary embodiment of the present specification, R7 and R8 can be the same substituent.

In an exemplary embodiment of the present specification, R1 to R8 are the same as or different from each other, and can be each independently an alkyl group which is unsubstituted or substituted with a halogen group, or an aryl group which is unsubstituted or substituted with a halogen group.

In an exemplary embodiment of the present specification, R1 to R8 are the same as or different from each other, and can be each independently an alkyl group having 1 to 30 carbon atoms, which is unsubstituted or substituted with a halogen group, or an aryl group having 6 to 50 carbon atoms, which is unsubstituted or substituted with a halogen group.

In an exemplary embodiment of the present specification, R1 to R8 are the same as or different from each other, and can be each independently an alkyl group having 1 to 10 carbon atoms, which is unsubstituted or substituted with a halogen group, or an aryl group having 6 to 20 carbon atoms, which is unsubstituted or substituted with a halogen group.

In an exemplary embodiment of the present specification, R1 to R8 are the same as or different from each other, and can be each independently an alkyl group having 1 to 10 carbon atoms, which is unsubstituted or substituted with a halogen group.

In an exemplary embodiment of the present specification, R1 to R8 are the same as or different from each other, and can be each independently an alkyl group having 1 to 5 carbon atoms, which is unsubstituted or substituted with a halogen group.

In an exemplary embodiment of the present specification, R1 to R8 are the same as or different from each other, and can be each independently a substituent of the following Chemical Formula 5 or 6:

[Chemical Formula 5] - (CH₂)ₗ(CF₂)ₘ(CF₃)ₙ(CH₃)₁₋ₙ

[

Chemical Formula 6] -C(H)ₐ((CH₂)ₗ(CF₂)ₘCF₃)₃₋ₐ

wherein in Chemical Formulae 5 and 6, l and m are each an integer from 0 to 10, and n and a are each 0 or 1.

In an exemplary embodiment of the present specification, R1 to R8 are the same as or different from each other, and can be each independently the substituent of Chemical Formula 5.

In an exemplary embodiment of the present specification, R1 to R8 are the same as or different from each other, and can be each independently -CF₃, -CH₂CH₃ or -CH₃.

In an exemplary embodiment of the present specification, the organic compound which can be hydrolyzed by the heavy water can include at least one of trifluoromethanesulfonic anhydride, trifluoroacetic anhydride, acetic anhydride, methanesulfonic anhydride, methyl acetate, ethyl acetate and dimethylacetamide.

In an exemplary embodiment of the present specification, the organic compound which can be hydrolyzed by the heavy water can include trifluoromethanesulfonic anhydride.

In an exemplary embodiment of the present specification, the organic compound which can be hydrolyzed by the heavy water can include trifluoroacetic anhydride.

In an exemplary embodiment of the present specification, the organic compound which can be hydrolyzed by the heavy water can include acetic anhydride.

In an exemplary embodiment of the present specification, the organic compound which can be hydrolyzed by the heavy water can include methanesulfonic anhydride.

In an exemplary embodiment of the present specification, the organic compound which can be hydrolyzed by the heavy water can include trifluoromethanesulfonic anhydride and trifluoroacetic anhydride.

In an exemplary embodiment of the present specification, the organic compound which can be hydrolyzed by the heavy water can include trifluoromethanesulfonic anhydride and acetic anhydride.

In an exemplary embodiment of the present specification, the organic compound which can be hydrolyzed by the heavy water can include methanesulfonic anhydride and trifluoroacetic anhydride.

In an exemplary embodiment of the present specification, the organic compound which can be hydrolyzed by the heavy water can include methanesulfonic anhydride and acetic anhydride.

In an exemplary embodiment of the present specification, the organic compound which can be hydrolyzed by the heavy water can include at least one of the compound of Chemical Formula 1 and the compound of Chemical Formula 2. When at least one of the compound of Chemical Formula 1 and the compound of Chemical Formula 2 is introduced into heavy water, hydrolysis with heavy water easily occurs even at room temperature.

In an exemplary embodiment of the present specification, the organic compound which can be hydrolyzed by the heavy water includes at least one of the compound of Chemical Formula 1 and the compound of Chemical Formula 2, and can further include at least one of the compound of Chemical Formula 3 and the compound of Chemical Formula 4. When the organic compound which can be hydrolyzed by the heavy water includes at least one of the compound of Chemical Formula 1 and the compound of Chemical Formula 2, it is possible to control a temperature occurring due to a hydrolysis reaction which is an exothermic reaction by adding at least one of the compound of Chemical Formula 3 and the compound of Chemical Formula 4 having a relatively slow hydrolysis reaction.

In an exemplary embodiment of the present specification, when the organic compound which can be hydrolyzed by the heavy water includes at least one of the compound of Chemical Formula 3 and the compound of Chemical Formula 4, the organic compound can further include at least one of the compound of Chemical Formula 1 and the compound of Chemical Formula 2. The hydrolysis reaction can be accelerated by adding the compound of Chemical Formulas 1 and the compound of Chemical Formula 2, in which the hydrolysis reaction relatively easily occurs.

In an exemplary embodiment of the present specification, the organic compound which can be hydrolyzed by the heavy water includes at least one of trifluoromethanesulfonic anhydride, trifluoroacetic anhydride, acetic anhydride and methanesulfonic anhydride, and can further include at least one of methyl acetate, ethyl acetate and dimethylacetamide.

In the organic compound which can be hydrolyzed by the heavy water, a weight ratio of at least one of the compound of Chemical Formula 3 and the compound of Chemical Formula 4 to at least one of the compound of Chemical Formula 1 and the compound of Chemical Formula 2 can be 100:0 to 0:100, 99:1 to 0:100, 90:10 to 0:100, 80:20 to 0:100, 70:30 to 0:100, 60:40 to 0:100, 50:50 to 0:100, 40:60 to 0:100, 30:70 to 0:100, 20:80 to 0:100, or 10:90 to 0:100.

According to an exemplary embodiment of the present specification, a content of the organic compound which can be hydrolyzed by the heavy water can be 1 mol% or more and 100 mol% or less based on the mole of heavy water. Specifically, according to an exemplary embodiment of the present specification, a content of the organic compound which can be hydrolyzed by the heavy water is not more than the molar equivalent of the heavy water, and is adjusted according to a target material. In this case, there is an advantage in that it is possible to increase the affinity between the aromatic compound and heavy water which are immiscible with each other and a deuterium substitution reactivity is enhanced.

According to an exemplary embodiment of the present specification, the solution can further include an organic solvent. The organic solvent is not particularly limited as long as the organic solvent can dissolve the aromatic compound, and can be selected according to the aromatic compound used.

The higher affinity the organic solvent has with heavy water, the better the organic solvent is, and even though the organic solvent does not have high affinity with heavy water, the organic solvent is not particularly limited as long as the organic solvent can be mixed with the organic compound which can be hydrolyzed.

When the solution further includes a deuterated aromatic solvent such as toluene-d8 as an additional deuterium source, an additional organic solvent may not be added.

According to an exemplary embodiment of the present specification, the solution is characterized by the fact that the solution does not contain a metal catalyst and an organic compound which can be hydrolyzed by heavy water plays the role thereof instead of the metal catalyst. Through this, problems caused by adding a metal catalyst, for example, the fact that hydrogen gas needs to be supplied, the fact that impurities due to hydrogen gas need to be removed, a fact that the process equipment capable of maintaining and withstanding a high reaction temperature and a high pressure needs to be provided, and the like are solved.

According to an exemplary embodiment of the present specification, the solution includes heavy water.

According to an exemplary embodiment of the present specification, the content of heavy water can be 0.1 times or more and 30 times or less of the weight of the aromatic compound. In this case, there is an advantage in that deuterium can be efficiently substituted from heavy water.

According to an exemplary embodiment of the present specification, the solution can include an additional deuterium source as well as heavy water. The deuterium source can be a deuterated aromatic solvent, for example, benzene-d6, toluene-d8, and the like.

According to an exemplary embodiment of the present specification, the content of the additional deuterium source can be 0.1 times or more and 30 times or less of the weight of the aromatic compound. In this case, there is an advantage in that the reactivity can be enhanced and the heat generation during the reaction can be reduced.

In an exemplary embodiment of the present specification, the aromatic compound is an aromatic compound including one or more aromatic rings, and specifically, is an aromatic compound including 1 to 30 aromatic rings. In this case, the meaning of having one or more aromatic rings means that there can be one or more aromatic rings of a monocyclic ring, a polycyclic ring, or a combination thereof, or there can be one or more aromatic rings (for example, a benzene ring) which are a basic unit. For example, the anthracene ring means one aromatic ring, or can mean that three benzene rings are linked based on a benzene ring which is a basic unit.

According to an exemplary embodiment of the present specification, the content of the aromatic compound can be 3 wt% or more and 50 wt% or less based on the total weight of the solvent.

In an exemplary embodiment of the present specification, the aromatic ring can be a substituted or unsubstituted, monocyclic or polycyclic hydrocarbon aromatic rings, or a substituted or unsubstituted, monocyclic or polycyclic heteroaromatic ring. For example, the aromatic ring can be a substituted or unsubstituted benzene ring, a substituted or unsubstituted naphthalene ring, a substituted or unsubstituted anthracene ring, a substituted or unsubstituted triphenylene ring, a substituted or unsubstituted phenanthrene ring, a substituted or unsubstituted dibenzofuran ring, a substituted or unsubstituted dibenzothiophene ring, a substituted or unsubstituted carbazole ring, and the like.

In an exemplary embodiment of the present specification, the aromatic compound can be a carbazole-based compound or an anthracene-based compound.

In an exemplary embodiment of the present specification, the aromatic compound can be a carbazole-based compound, and specifically, can be a substituted or unsubstituted carbazole, or a substituted or unsubstituted carbazole having an additional ring to which an adjacent group is bonded.

The carbazole having an additional ring to which an adjacent group is bonded can be a substituted or unsubstituted benzocarbazole, a substituted or unsubstituted dibenzocarbazole, a substituted or unsubstituted furocarbazole, or a substituted or unsubstituted indolocarbazole.

In an exemplary embodiment of the present specification, the aromatic compound can be benzene, toluene, naphthalene, naphthylamine, indole, carbazole, anthraquinone, or dihydroindolocarbazole.

In an exemplary embodiment of the present specification, the aromatic compound can be an anthracene-based compound, and specifically, can be a substituted or unsubstituted anthracene.

In an exemplary embodiment of the present specification, the aromatic compound can include a compound of the following Chemical Formula A: 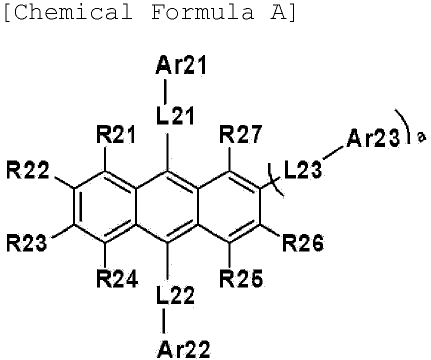 wherein in Chemical Formula A:
L21 to L23 are the same as or different from each other, and are each independently a direct bond or a substituted or unsubstituted arylene group, or a substituted or unsubstituted heteroarylene group;
R21 to R27 are the same as or different from each other, and are each independently hydrogen, a substituted or unsubstituted alkyl group, a substituted or unsubstituted cycloalkyl group; a substituted or unsubstituted silyl group, a substituted or unsubstituted aryl group, or a substituted or unsubstituted heteroaryl group;
Ar21 to Ar23 are the same as or different from each other, and are each independently a substituted or unsubstituted aryl group or a substituted or unsubstituted heteroaryl group; and
a is 0 or 1.

In an exemplary embodiment of the present specification, the aromatic compound participating in the deuterated reaction can be any one of the following Chemical Formulae 7 to 11. By the deuterated reaction, at least one hydrogen of the selected compounds is substituted with deuterium.

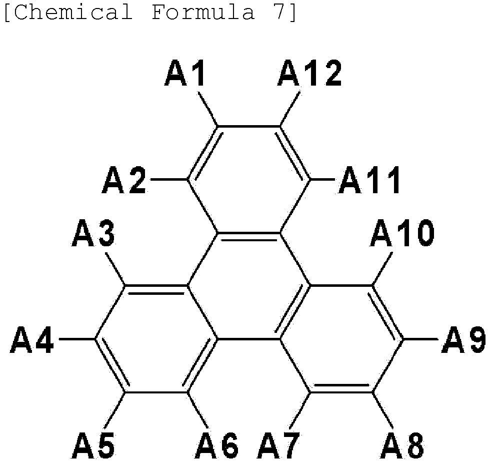

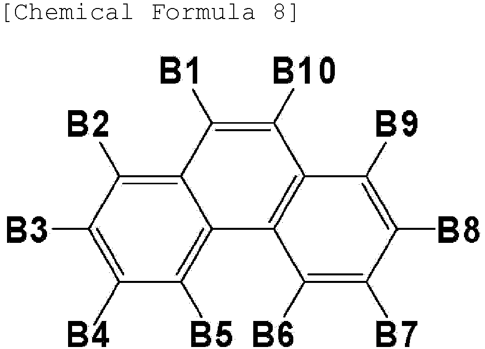

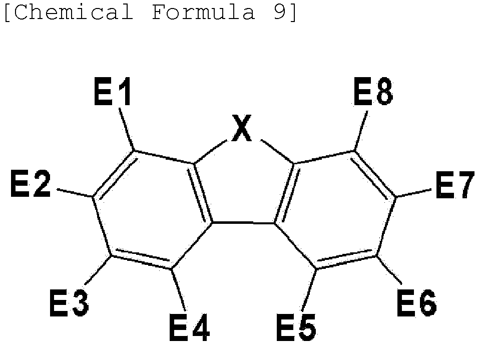

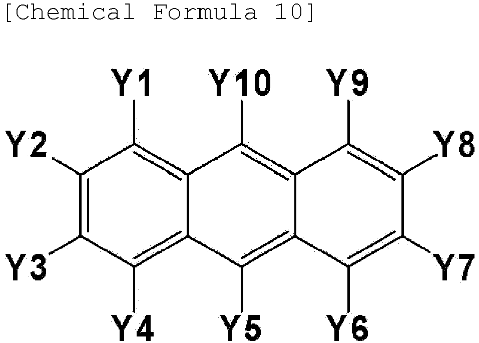

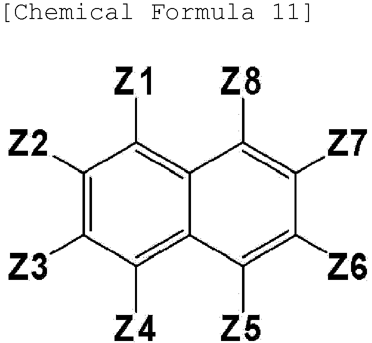

In Chemical Formulae 7 to 11:
X is O, S or NR;
R is hydrogen, deuterium, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group;
A1 to A12 are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
B1 to B10 are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
E1 to E8 are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
Y1 to Y10 are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring; and
Z1 to Z8 are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring.

Examples of the substituents in the present specification will be described below, but are not limited thereto.

The term "substitution" means that a hydrogen atom bonded to a carbon atom of a compound is changed into another substituent, and a position to be substituted is not limited as long as the position is a position at which the hydrogen atom is substituted, that is, a position at which the substituent can be substituted, and when two or more are substituted, the two or more substituents can be the same as or different from each other.

In the present specification, the term "substituted or unsubstituted" means being substituted with one or two or more substituents selected from the group consisting of a halogen group, a cyano group, a nitro group, a hydroxy group, an amine group, a silyl group, a boron group, an alkoxy group, an alkyl group, a cycloalkyl group, an aryl group, and a heterocyclic group, being substituted with a substituent to which two or more substituents among the above-exemplified substituents are linked, or having no substituent. For example, "the substituent to which two or more substituents are linked" can be a biphenyl group. That is, the biphenyl group can also be an aryl group, and can be interpreted as a substituent to which two phenyl groups are linked.

In the present specification, the "adjacent" group can mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring can be interpreted as groups which are "adjacent" to each other. In addition, substituents (four in total) linked to two consecutive carbons in an aliphatic ring can be interpreted as "adjacent" groups.

In the present specification, the meaning "being bonded to an adjacent group to form a ring" among substituents means being bonded to an adjacent group to form a substituted or unsubstituted aliphatic hydrocarbon ring, a substituted or unsubstituted aromatic hydrocarbon ring; a substituted or unsubstituted aliphatic hetero ring, or a substituted or unsubstituted aromatic hetero ring.

In the present specification, "a five-membered or six-membered ring formed by bonding adjacent groups" means that a ring including a substituent participating in the ring formation is five-membered or six-membered. It is possible to include an additional ring fused to the ring including the substituent participating in the ring formation.

In the present specification, when being bonded to an adjacent group among substituents to form a ring, a ring to be formed can be any one of the following structures:

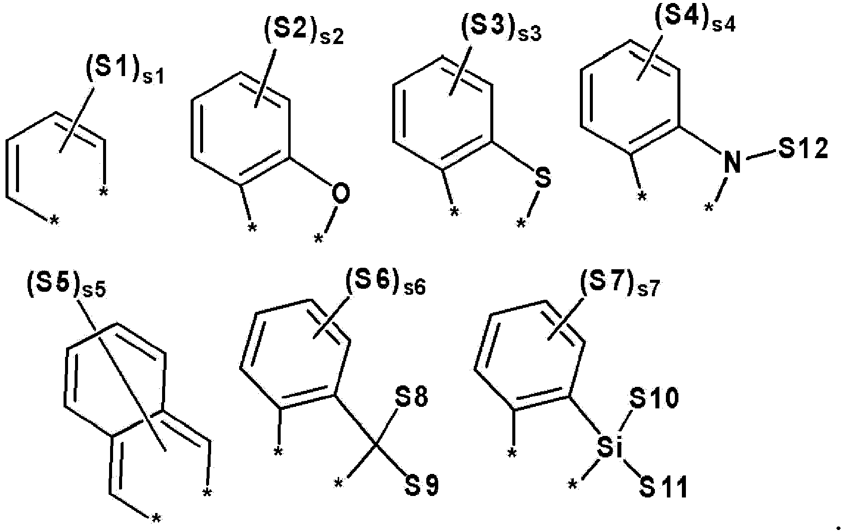

In the structures, S1 to S12 are each independently hydrogen, a leaving group, a hydroxyl group, a substituted or unsubstituted amine group, a cyano group, a substituted or unsubstituted alkyl group, a substituted or unsubstituted aryl group, or a substituted or unsubstituted heterocyclic group;
s1 to s4, s6, and s7 are each an integer from 1 to 4;
s5 is an integer from 1 to 6;
when s1 is 2 or higher, the S1s are the same as or different from each other;
   when s2 is 2 or higher, the S2s are the same as or different from each other;
when s3 is 2 or higher, the S3s are the same as or different from each other;
when s4 is 2 or higher, the S4s are the same as or different from each other;
when s5 is 2 or higher, the S5s are the same as or different from each other;
when s6 is 2 or higher, the S6s are the same as or different from each other; and
when s7 is 2 or higher, the S7s are the same as or different from each other.

In the present specification, S1 to S7 are hydrogen.

In the present specification, S8 to S11 are each independently a substituted or unsubstituted alkyl group or a substituted or unsubstituted aryl group.

In the present specification, S12 is a substituted or unsubstituted aryl group or a substituted or unsubstituted heterocyclic group.

In the present specification, examples of a halogen group include fluorine (-F), chlorine (-Cl), bromine (-Br) or iodine (-I).

In the present specification, a silyl group can be a chemical formula of -SiYₐY_{b}Y_{c}, and the Yₐ, Y_{b}, and Y_{c} can be each hydrogen, a substituted or unsubstituted alkyl group, or a substituted or unsubstituted aryl group. Specific examples of the silyl group include a trimethylsilyl group, a triethylsilyl group, a tertbutyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, a boron group can be a chemical formula of -BY_{d}Yₑ, and the Y_{d} and Yₑ can be each hydrogen, a substituted or unsubstituted alkyl group, or a substituted or unsubstituted aryl group. Specific examples of the boron group include a dimethylboron group, a diethylboron group, a tert-butylmethylboron group, a diphenylboron group, a phenylboron group, and the like, but are not limited thereto.

In the present specification, the alkyl group can be straight-chained or branched, and the number of carbon atoms thereof is not particularly limited, but is preferably 1 to 60. According to an exemplary embodiment, the number of carbon atoms of the alkyl group is 1 to 30. According to another exemplary embodiment, the number of carbon atoms of the alkyl group is 1 to 20. According to still another exemplary embodiment, the number of carbon atoms of the alkyl group is 1 to 10. Specific examples of the alkyl group include a methyl group, an ethyl group, a propyl group, an n-propyl group, an isopropyl group, a butyl group, an n-butyl group, an isobutyl group, a tert-butyl group, a pentyl group, an n-pentyl group, a hexyl group, an n-hexyl group, a heptyl group, an n-heptyl group, an octyl group, an n-octyl group, and the like, but are not limited thereto.

In the present specification, the alkoxy group can be straight-chained, branched, or cyclic. The number of carbon atoms of the alkoxy group is not particularly limited, but is preferably 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, and the like, but are not limited thereto.

Substituents including an alkyl group, an alkoxy group, and other alkyl group moieties described in the present specification include both a straight-chained form and a branched form.

In the present specification, a cycloalkyl group is not particularly limited, but has preferably 3 to 60 carbon atoms, and according to an exemplary embodiment, the number of carbon atoms of the cycloalkyl group is 3 to 30. According to another exemplary embodiment, the number of carbon atoms of the cycloalkyl group is 3 to 20. According to yet another exemplary embodiment, the number of carbon atoms of the cycloalkyl group is 3 to 6. Specific examples thereof include a cyclopropyl group, a cyclobutyl group, a cyclopentyl group, a cyclohexyl group, a cycloheptyl group, a cyclooctyl group, and the like, but are not limited thereto.

In the present specification, an aryl group is not particularly limited, but has preferably 6 to 60 carbon atoms, and can be a monocyclic aryl group or a polycyclic aryl group. According to an exemplary embodiment, the number of carbon atoms of the aryl group is 6 to 39. According to an exemplary embodiment, the number of carbon atoms of the aryl group is 6 to 30. Examples of the monocyclic aryl group include a phenyl group, a biphenyl group, a terphenyl group, a quaterphenyl group, and the like, but are not limited thereto. Examples of the polycyclic aryl group include a naphthyl group, an anthracenyl group, a phenanthrenyl group, a pyrenyl group, a perylenyl group, a triphenylenyl group, a chrysenyl group, a fluorenyl group, a triphenylenyl group, and the like, but are not limited thereto.

In the present specification, a fluorene group can be substituted, and two substituents can be bonded to each other to form a spiro structure.

When the fluorene group is substituted, the fluorene group can be a spirofluorene group such as 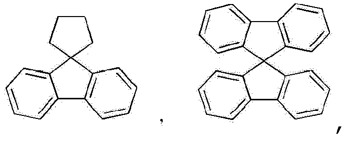 and a substituted fluorene group such as 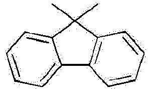 (a 9,9-dimethylfluorene group) and 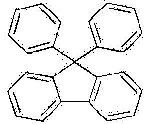 (a 9,9-diphenylfluorene group). However, the substituent is not limited thereto.

In the present specification, a heterocyclic group is a cyclic group including one or more of N, O, P, S, Si, and Se as a heteroatom, and the number of carbon atoms thereof is not particularly limited, but is preferably 2 to 60. According to an exemplary embodiment, the number of carbon atoms of the heterocyclic group is 2 to 36. Examples of the heterocyclic group include a pyridine group, a pyrrole group, a pyrimidine group, a quinoline group, a pyridazine group, a furan group, a thiophene group, an imidazole group, a pyrazole group, a dibenzofuran group, a dibenzothiophene group, a carbazole group, a benzocarbazole group, a benzonaphthofuran group, a benzonaphthothiophene group, an indenocarbazole group, an indolocarbazole group, and the like, but are not limited thereto.

In the present specification, the above-described description on the heterocyclic group can be applied to a heteroaryl group except for an aromatic heteroaryl group.

In the present specification, an amine group can be selected from the group consisting of -NH₂, an alkylamine group, an N-alkylarylamine group, an arylamine group, an N-arylheteroarylamine group, an N-alkylheteroarylamine group, and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, an N-phenylnaphthylamine group, a ditolylamine group, an N-phenyltolylamine group, an N-phenylbiphenylamine group, an N-phenylnaphthylamine group, an N-biphenylnaphthylamine group, an N-naphthylfluorenylamine group, an N-phenyl-phenanthrenylamine group, an N-biphenylphenanthrenyl-amine group, an N-phenylfluorenylamine group, an N-phenyl terphenylamine group, an N-phenanthrenyl-fluorenylamine group, an N-biphenylfluorenylamine group, and the like, but are not limited thereto.

In the present specification, an N-alkylarylamine group means an amine group in which an alkyl group and an aryl group are substituted with N of the amine group.

In the present specification, an N-arylheteroarylamine group means an amine group in which an aryl group and a heteroaryl group are substituted with N of the amine group.

In the present specification, an N-alkylheteroarylamine group means an amine group in which an alkyl group and a heteroaryl group are substituted with N of the amine group.

In the present specification, an alkyl group, an aryl group, and a heteroaryl group in an alkylamine group, an N-alkylarylamine group, an arylamine group, an N-arylheteroarylamine group, an N-alkylheteroarylamine group, and a heteroarylamine group are each the same as the above-described examples of the alkyl group, the aryl group, and the heteroaryl group.

In an exemplary embodiment of the present specification, the aromatic compound can be any one of the following compounds:

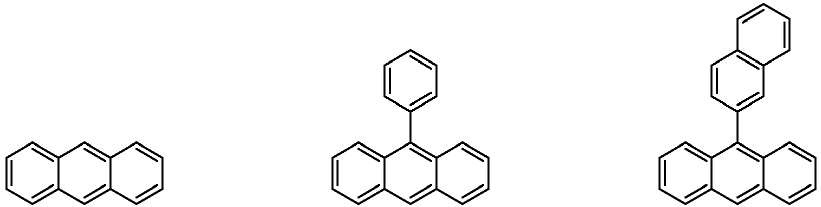

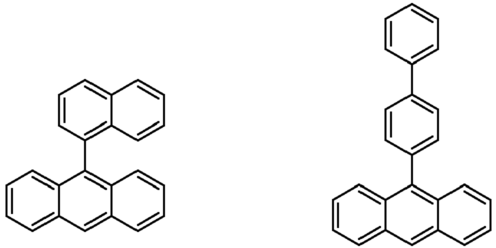

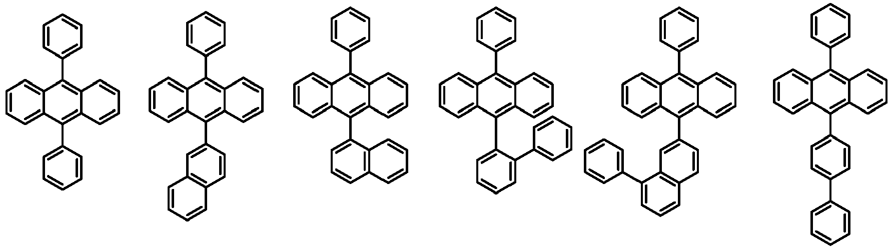

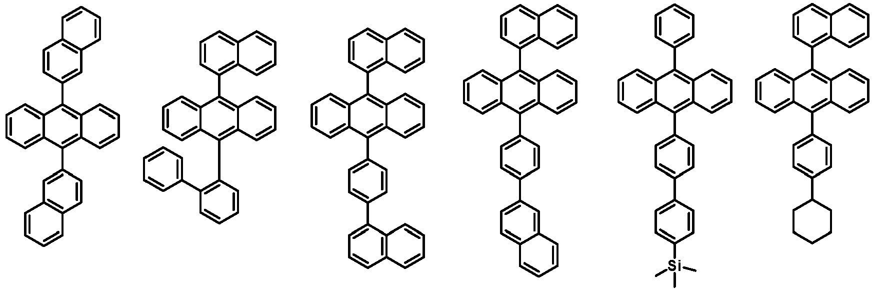

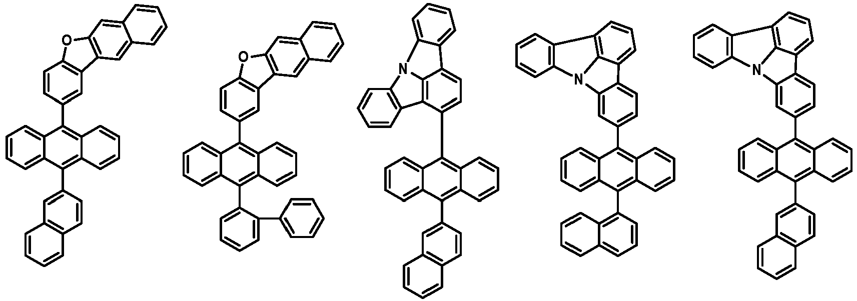

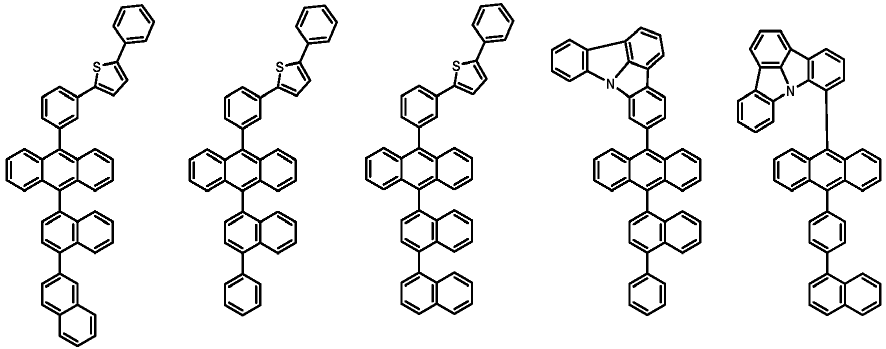

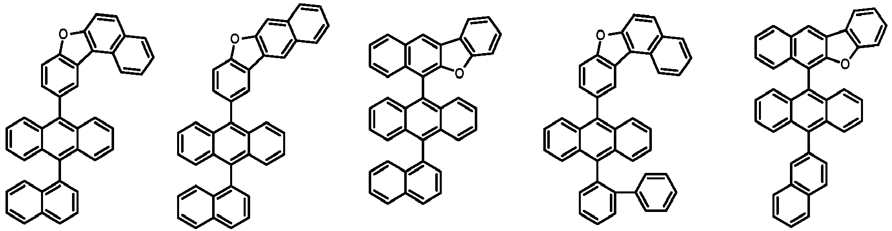

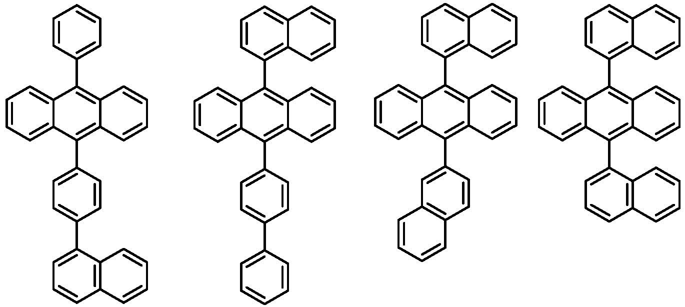

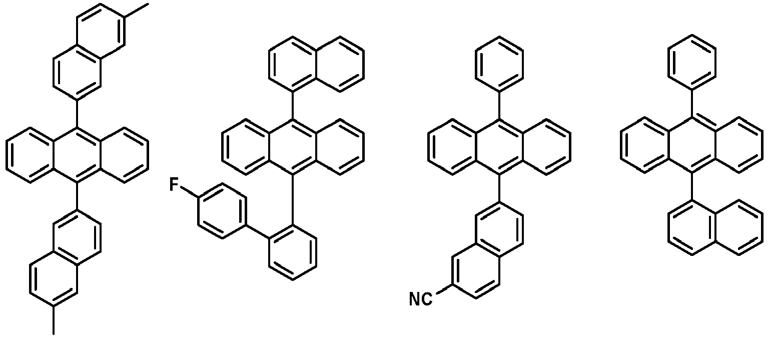

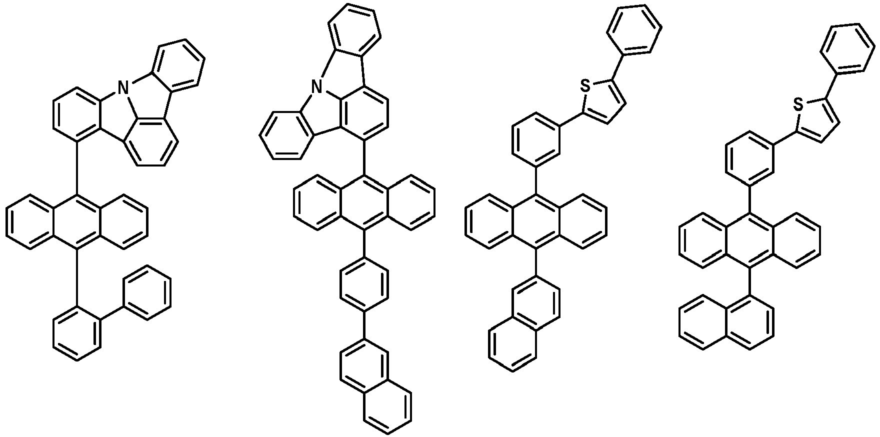

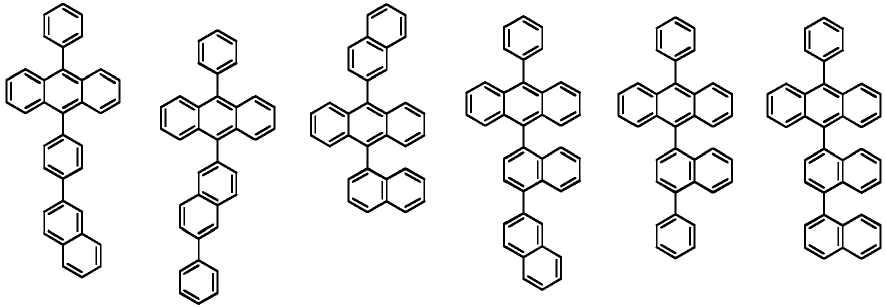

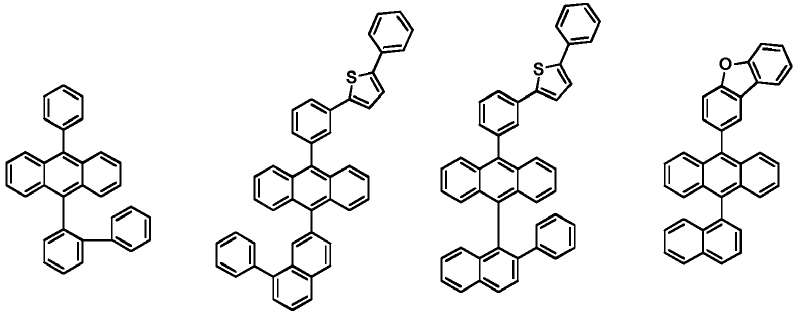

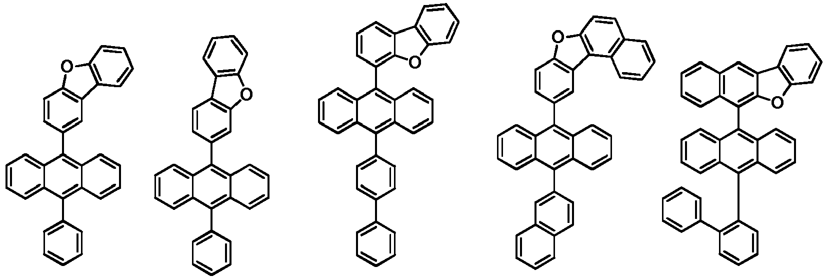

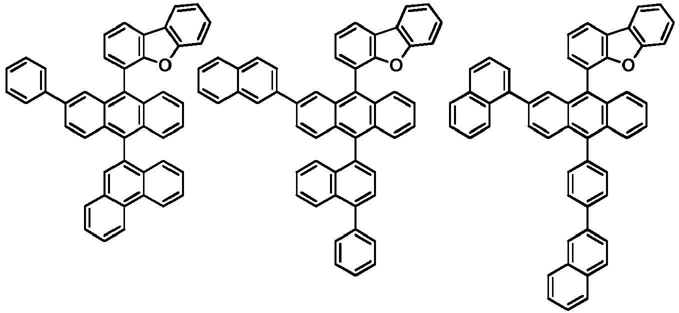

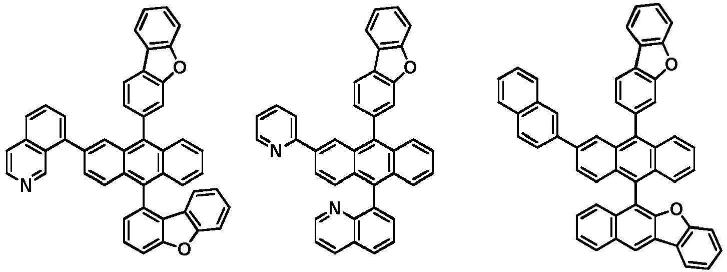

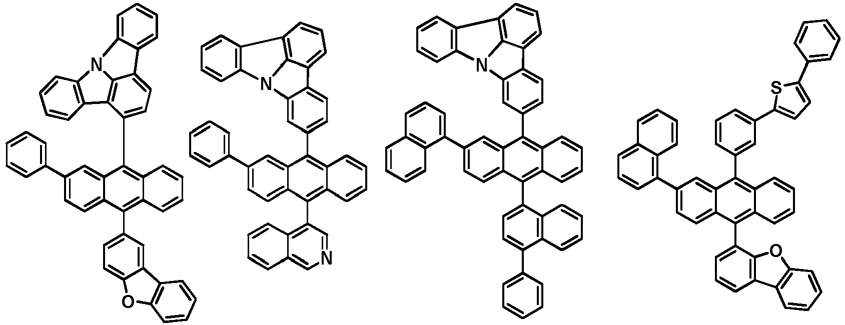

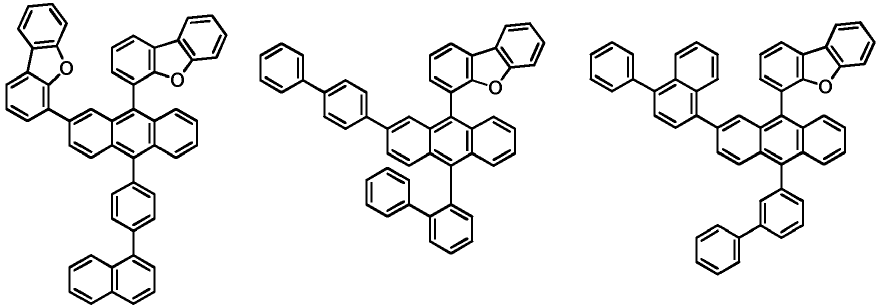

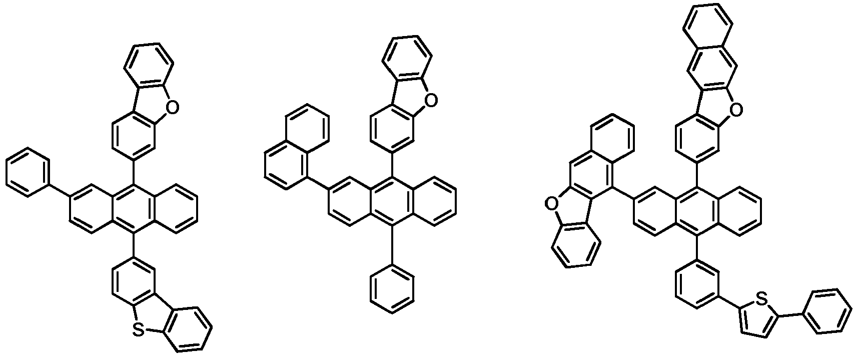

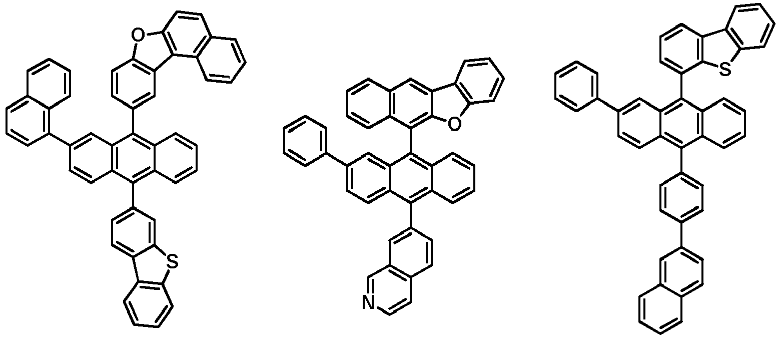

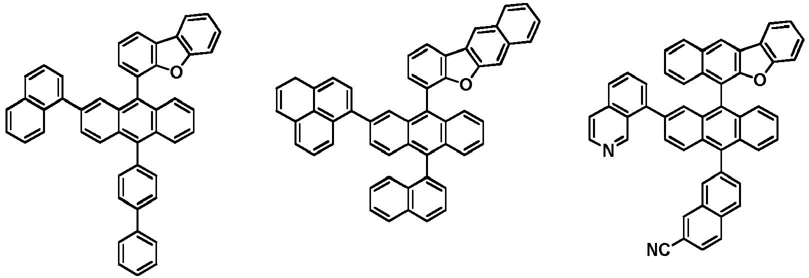

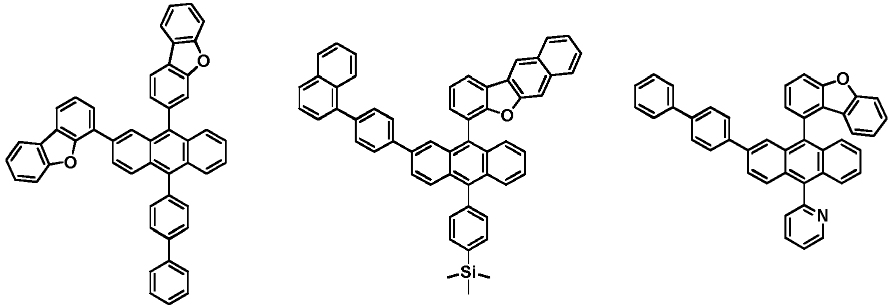

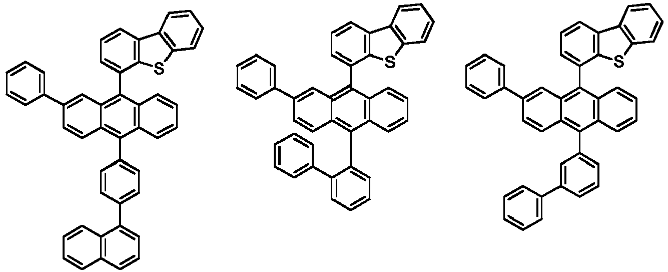

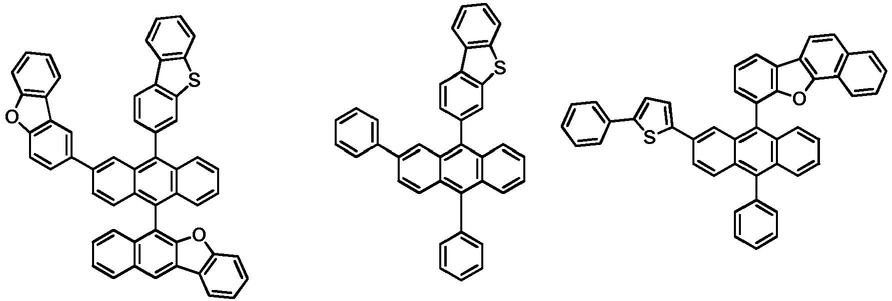

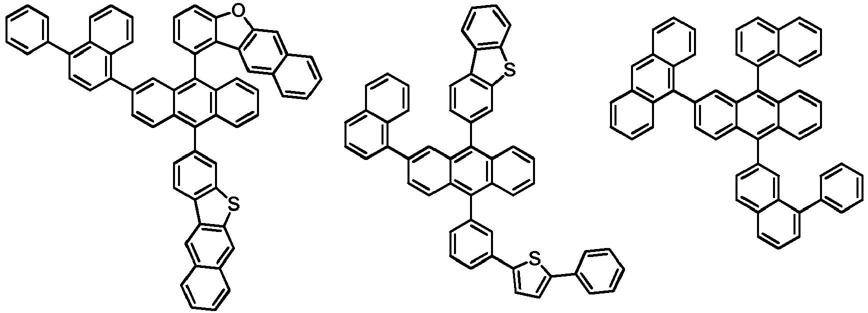

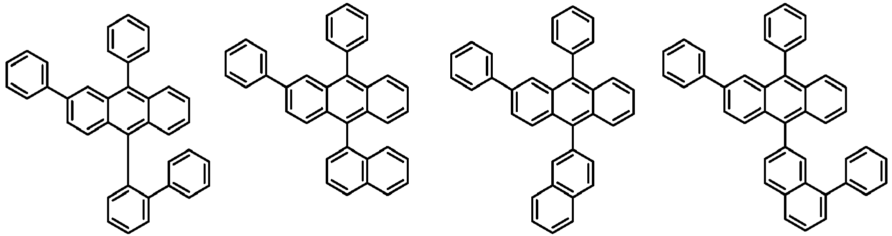

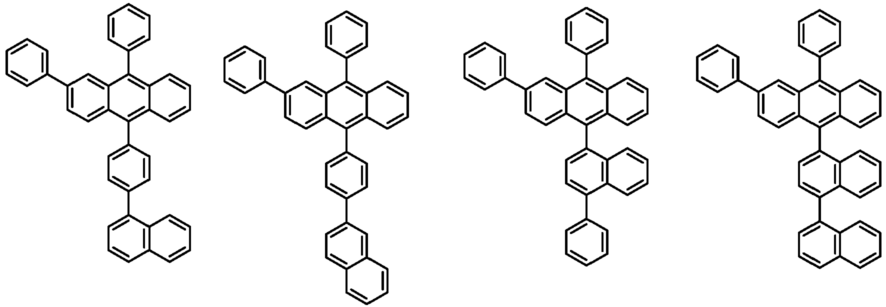

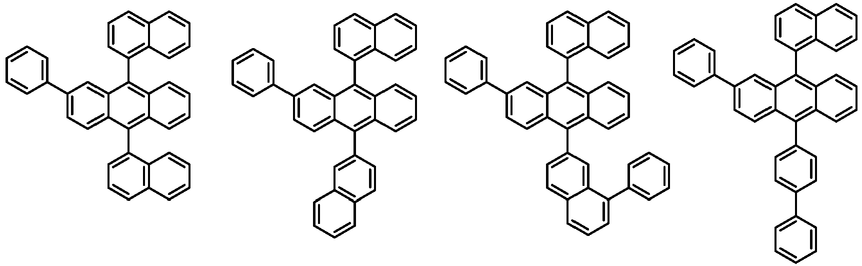

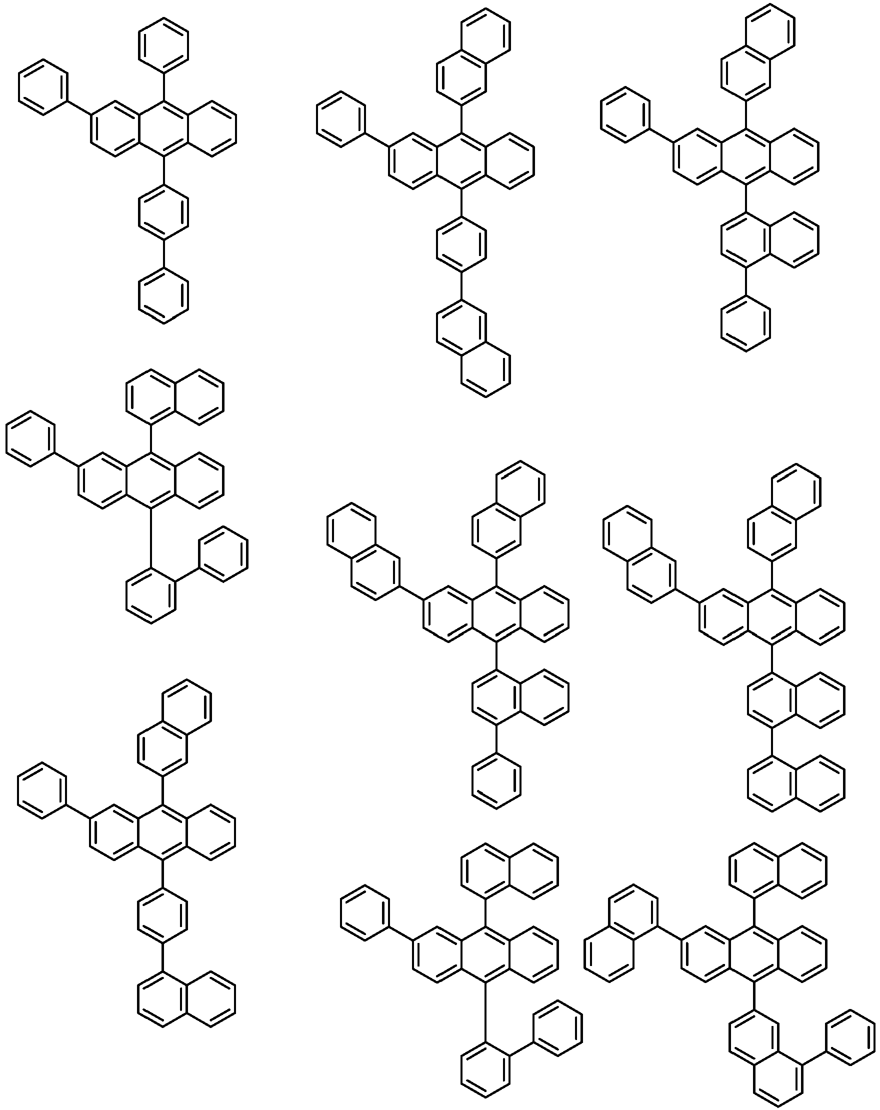

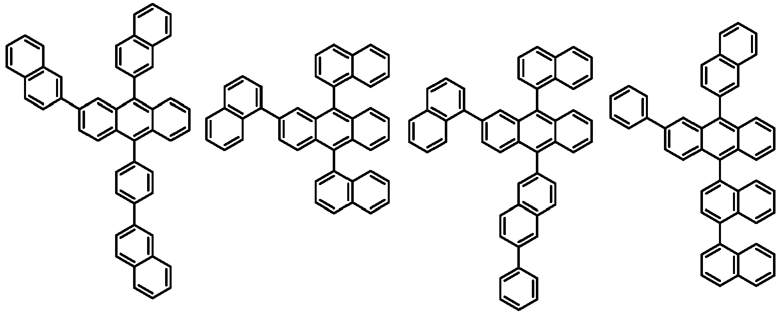

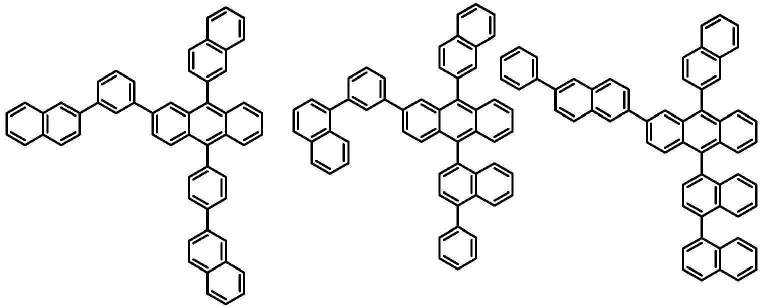

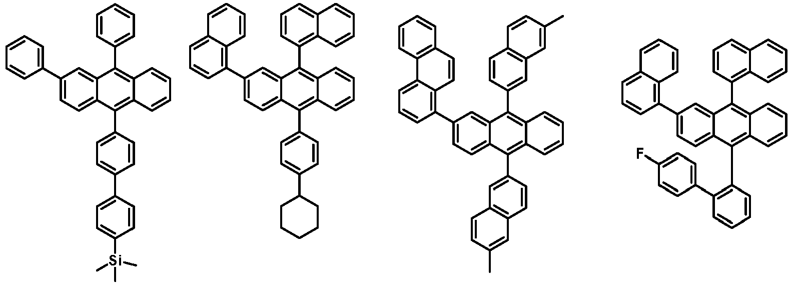

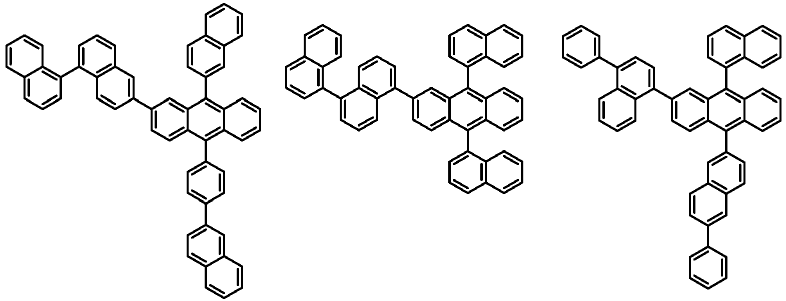

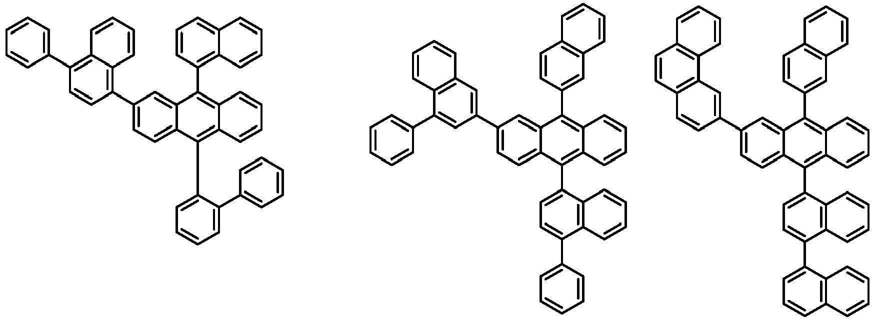

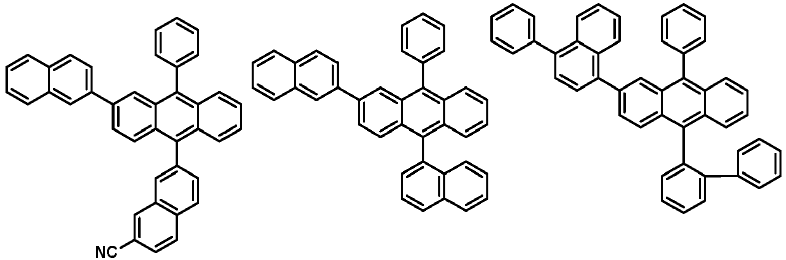

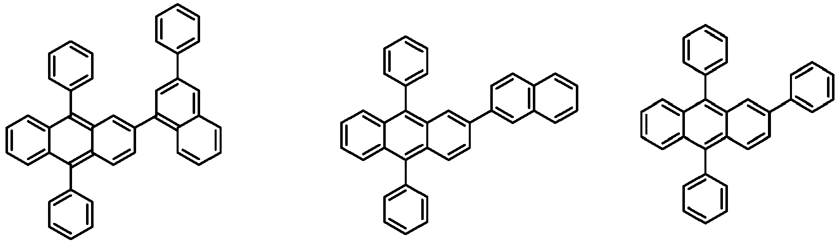

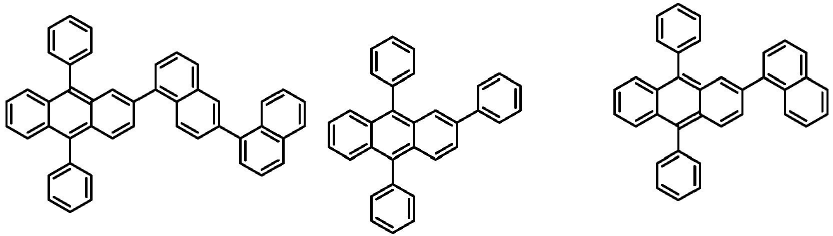

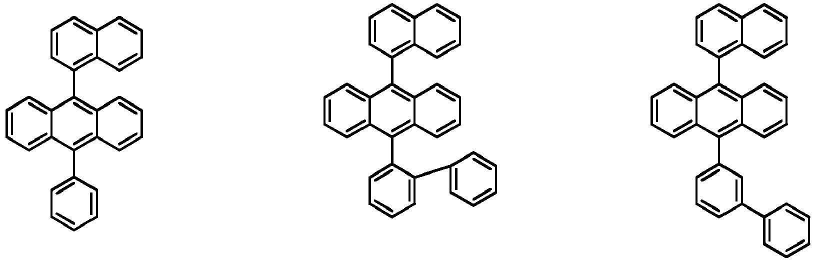

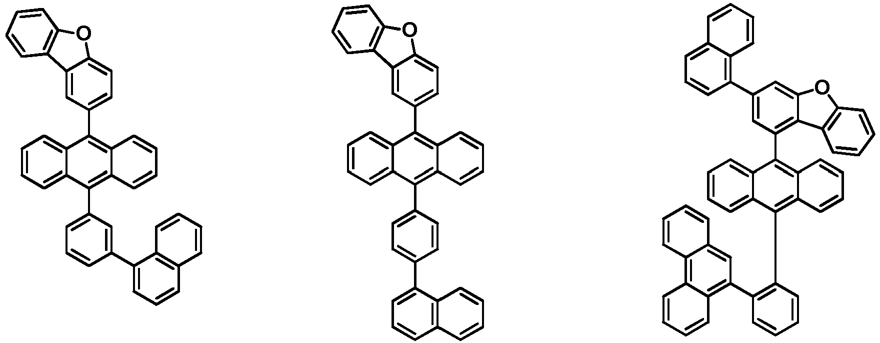

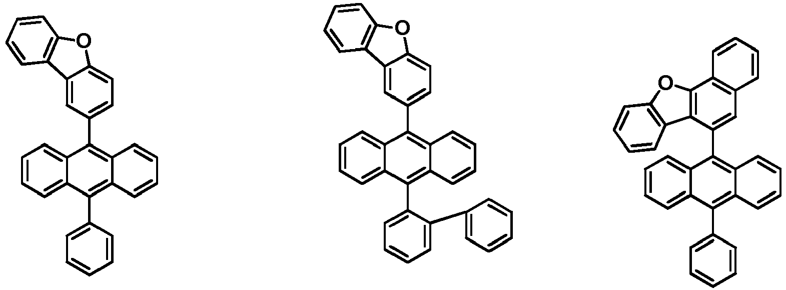

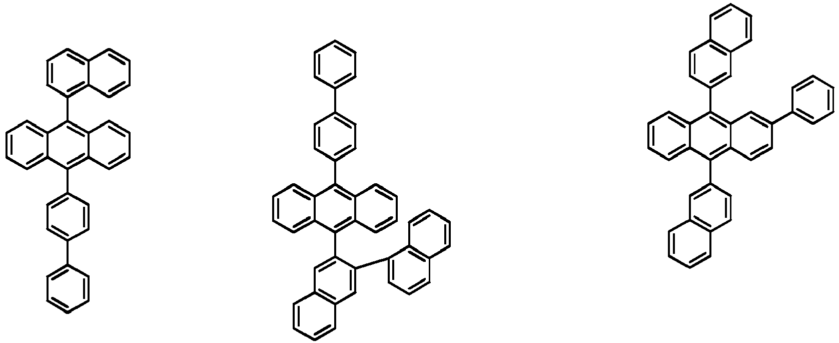

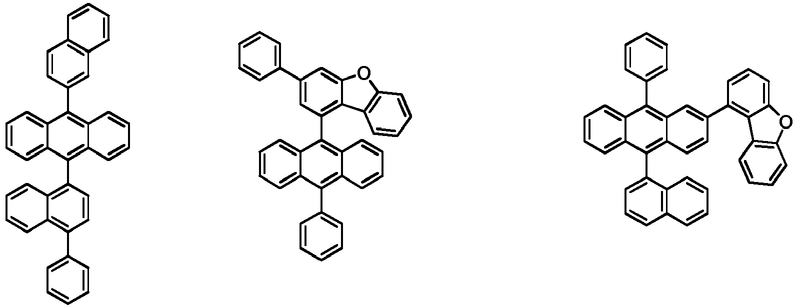

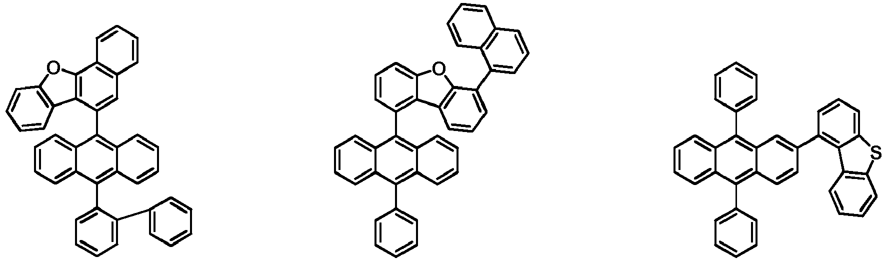

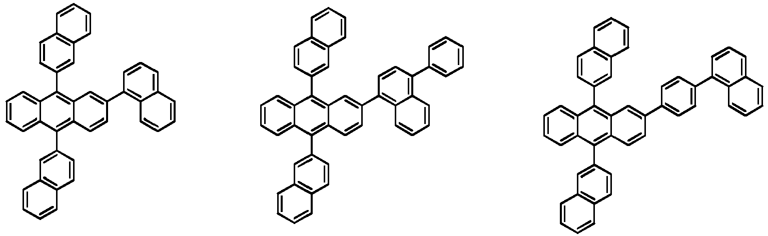

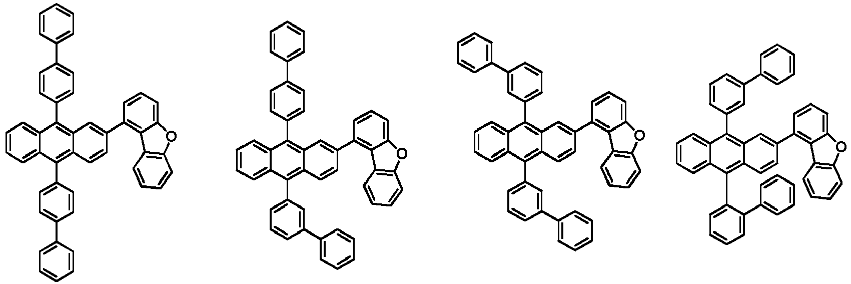

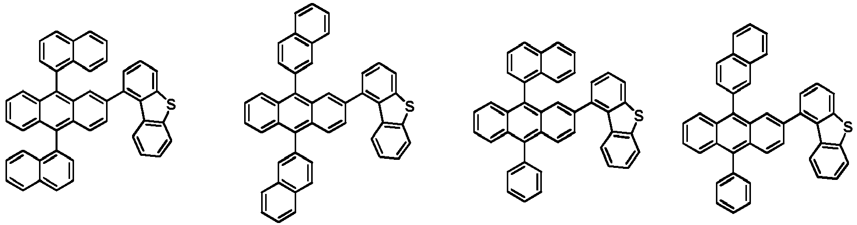

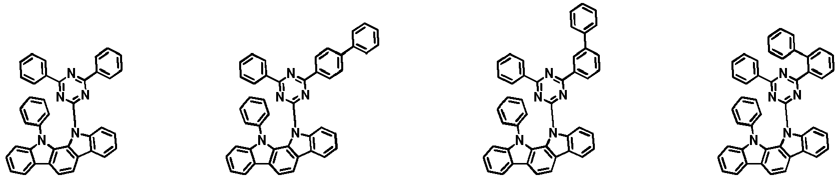

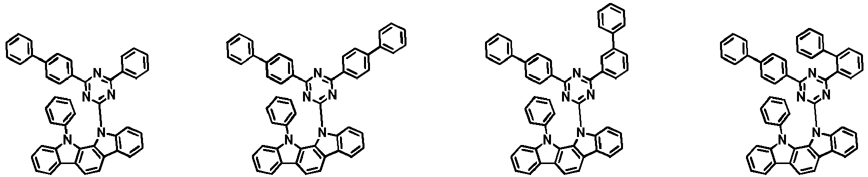

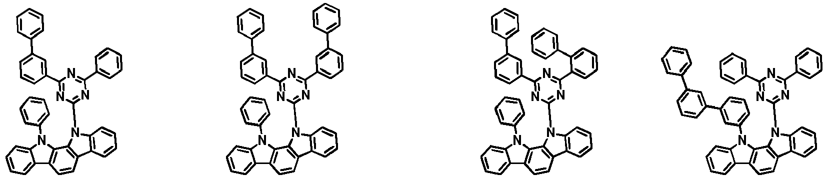

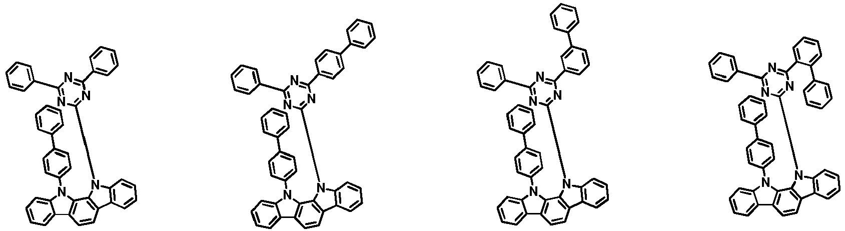

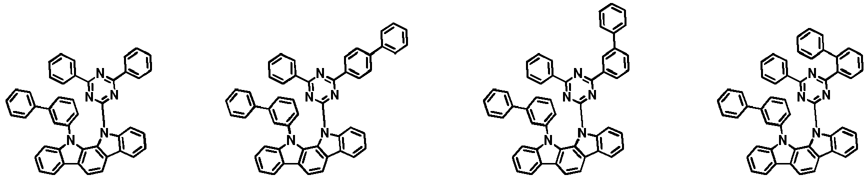

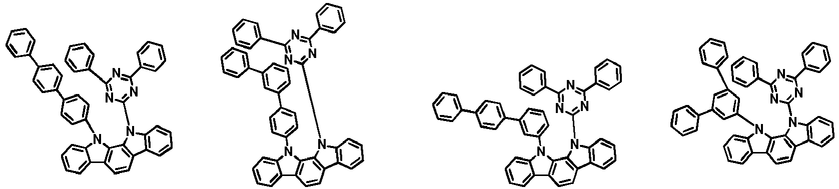

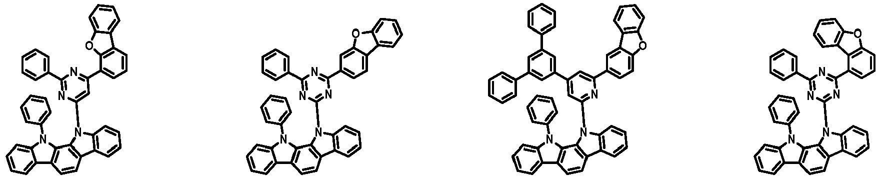

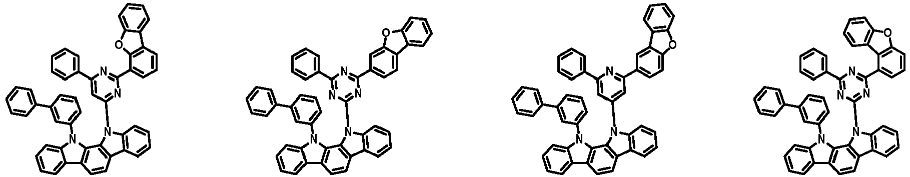

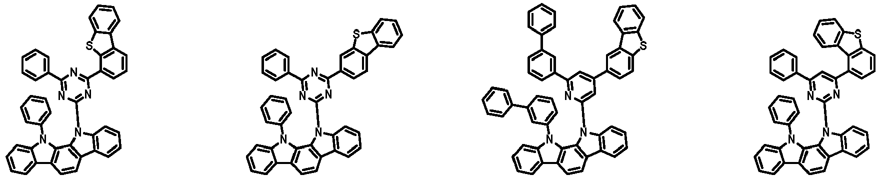

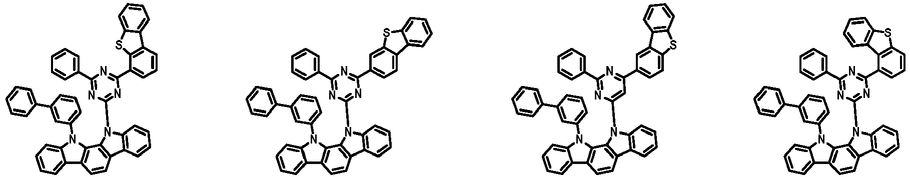

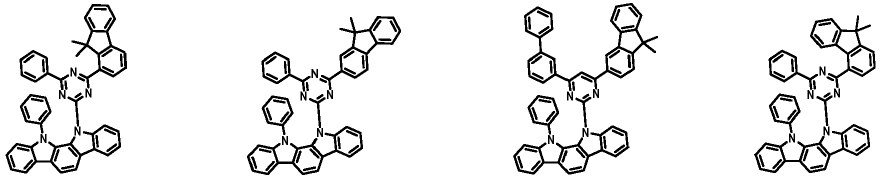

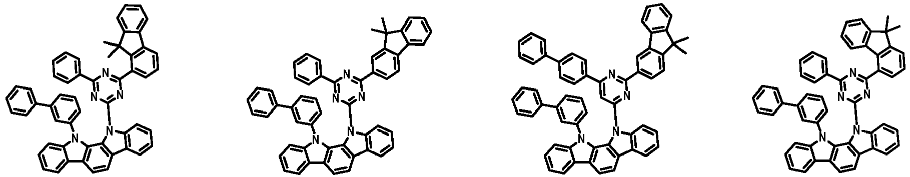

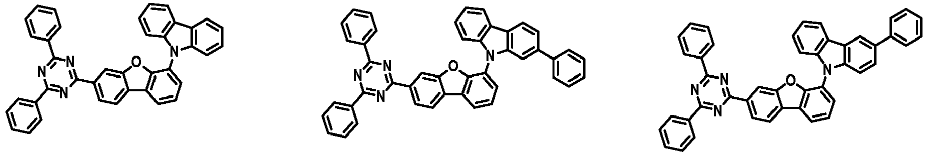

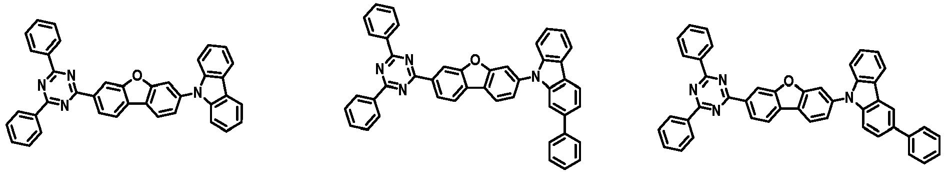

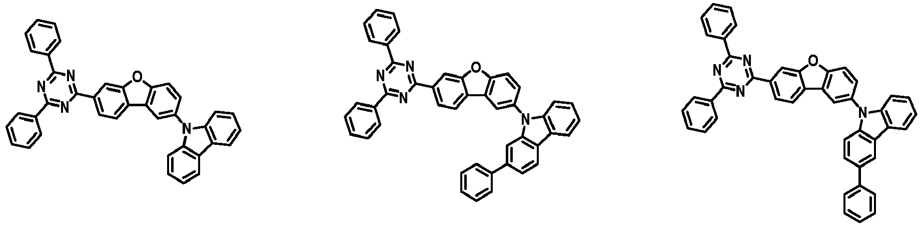

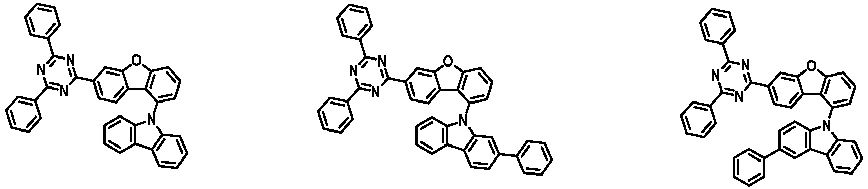

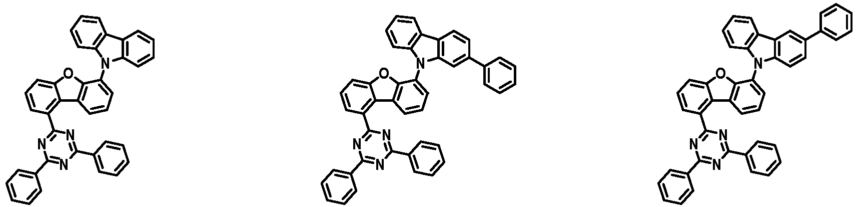

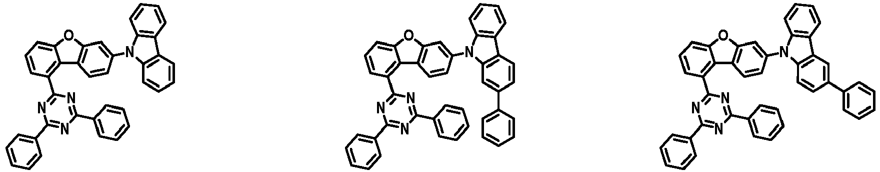

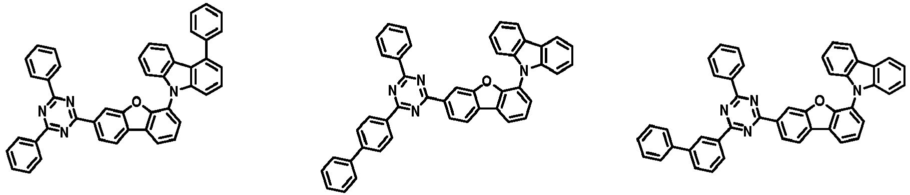

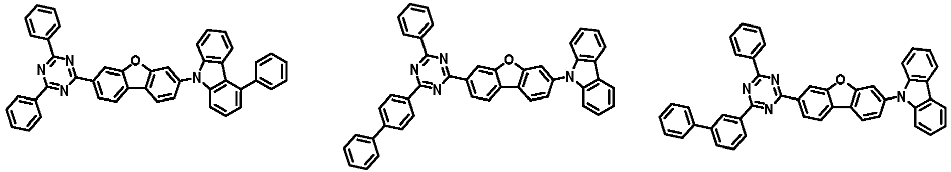

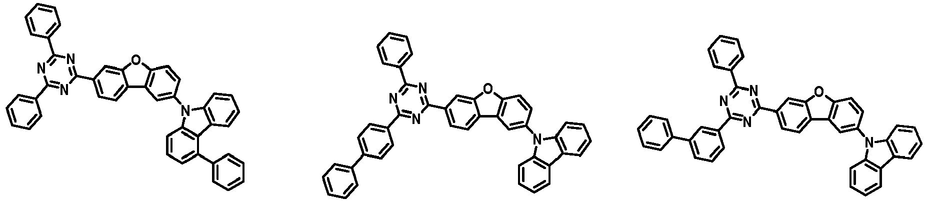

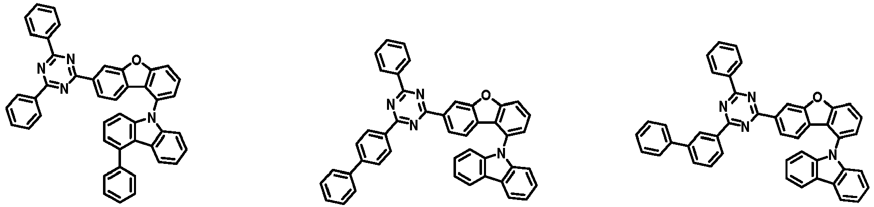

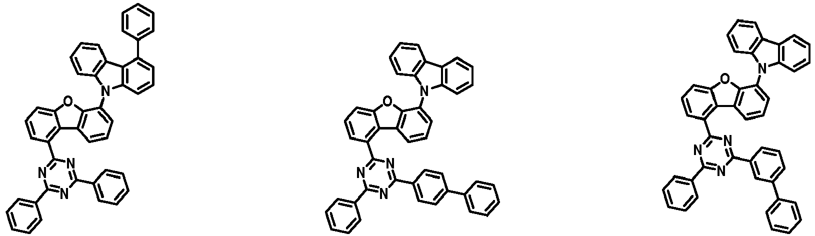

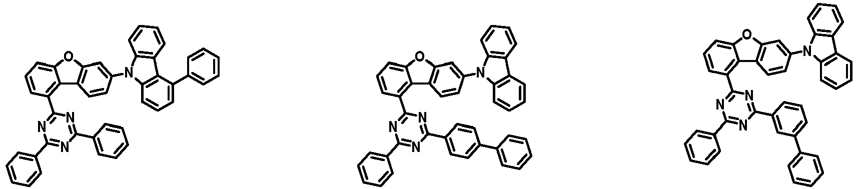

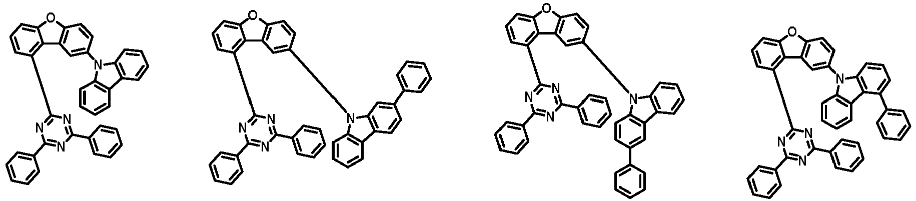

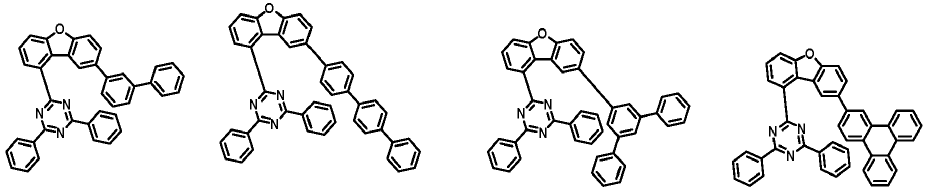

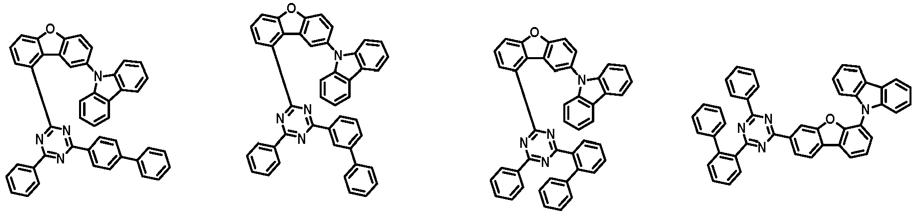

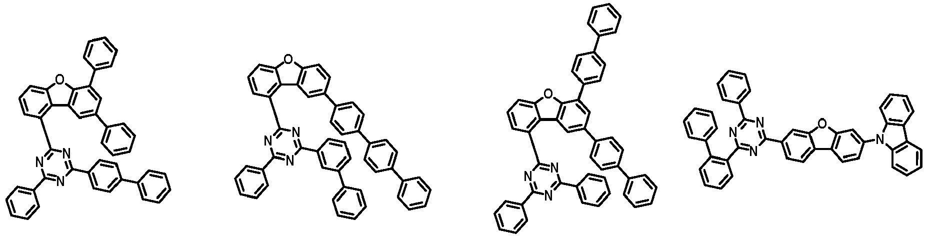

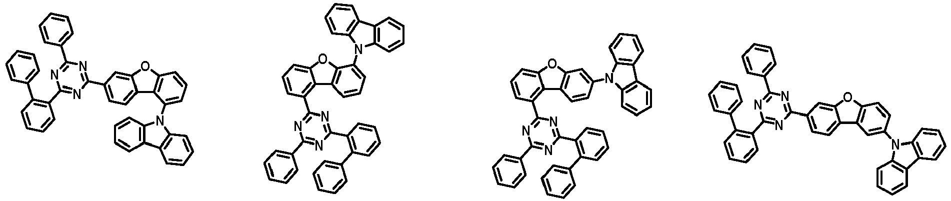

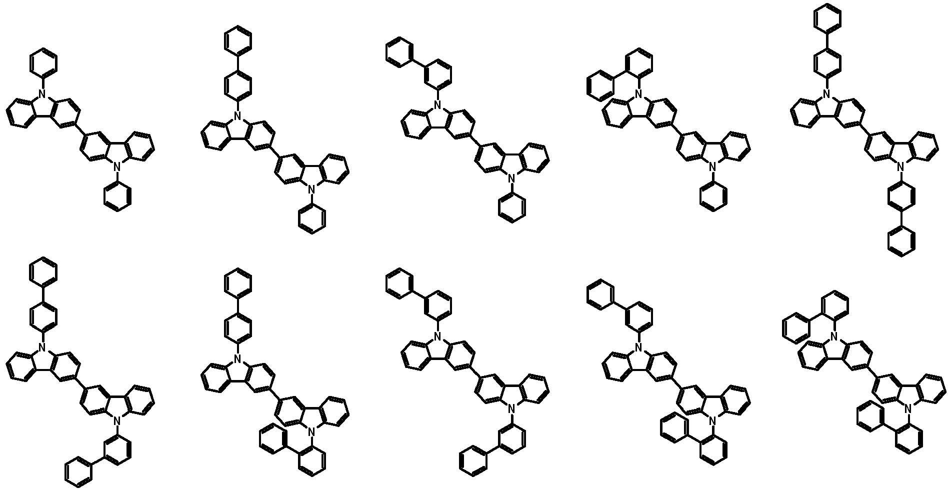

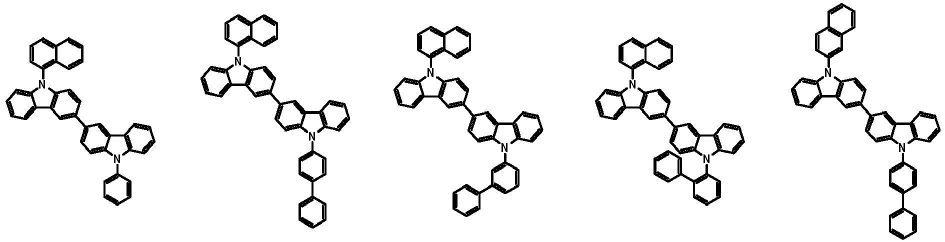

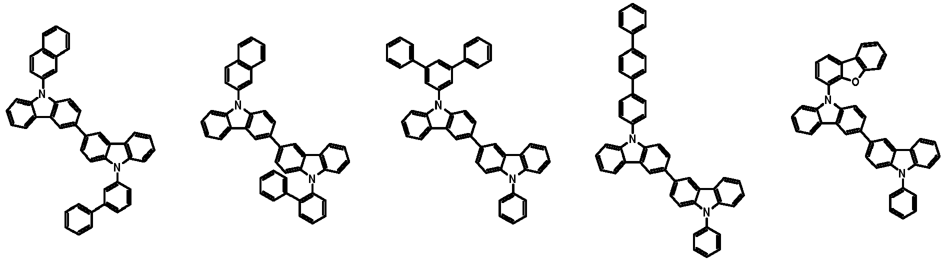

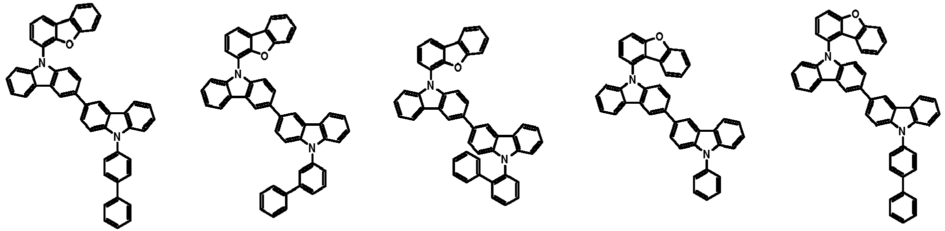

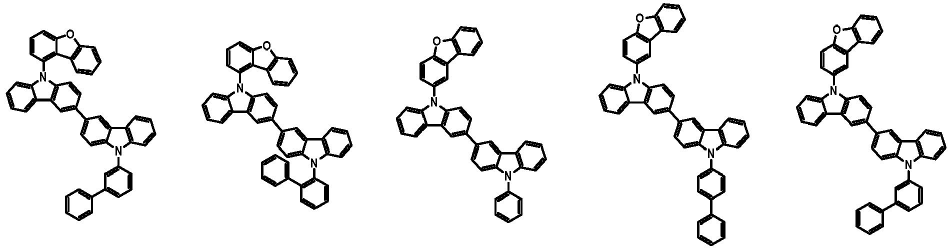

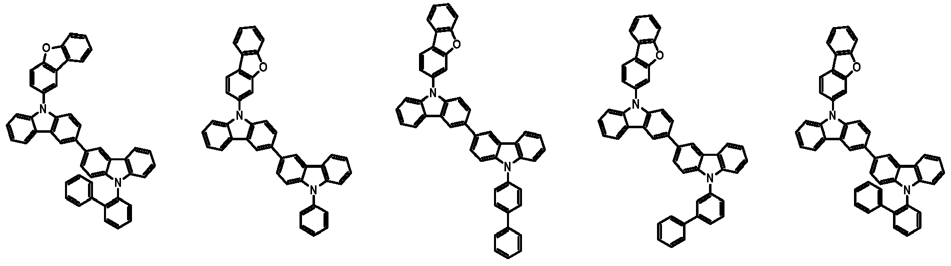

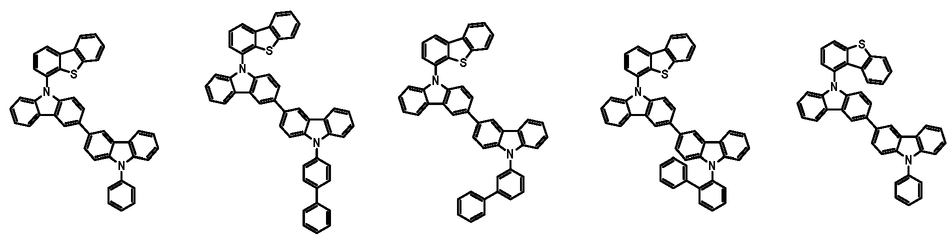

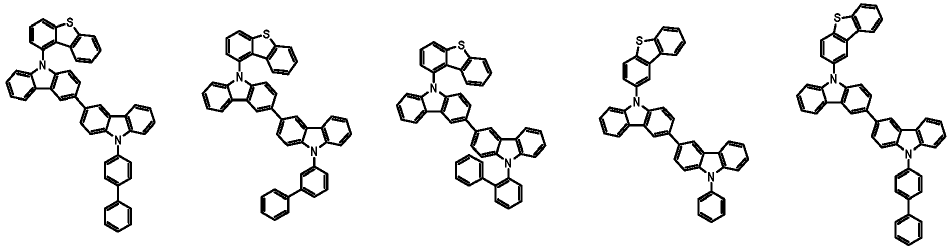

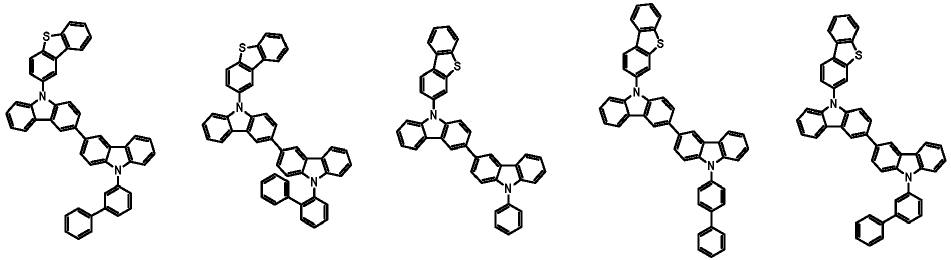

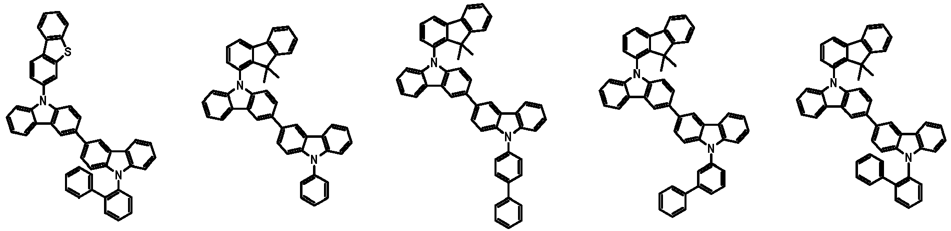

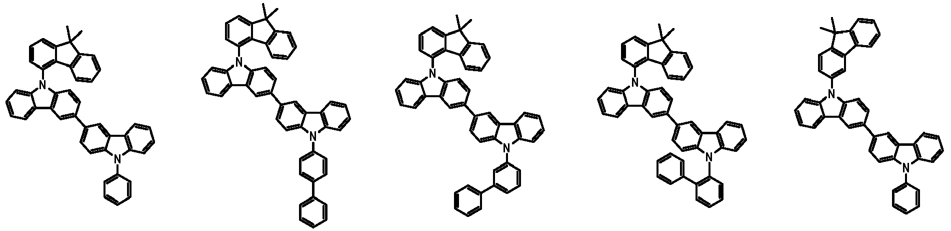

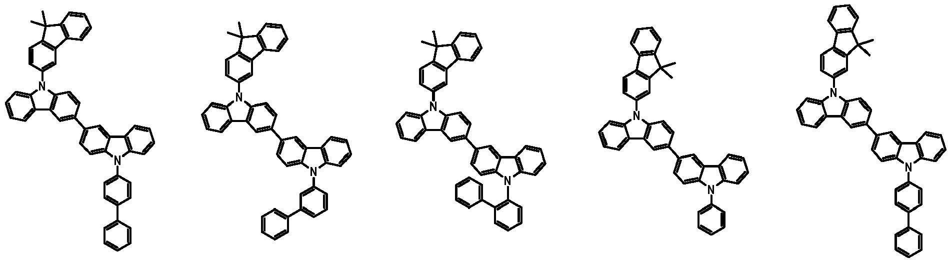

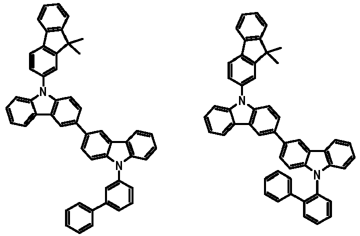

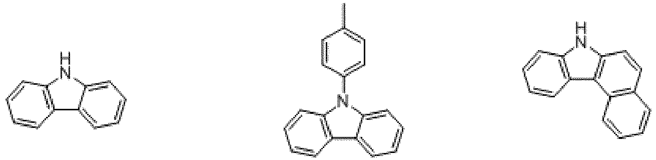

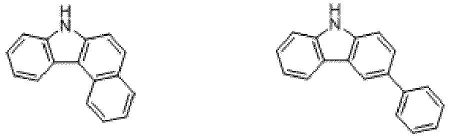

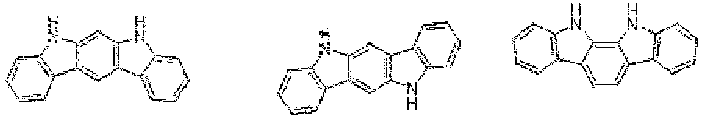

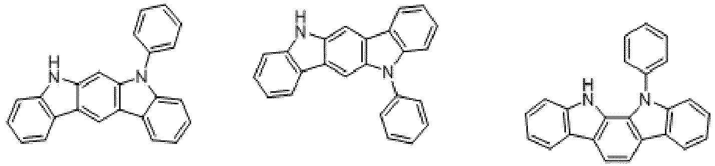

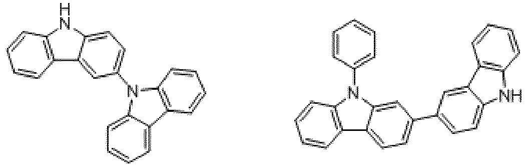

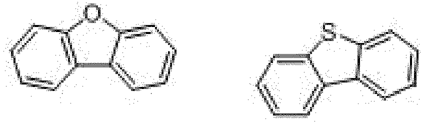

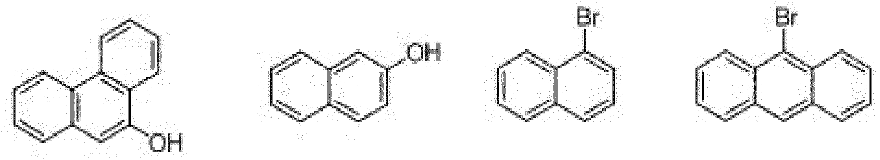

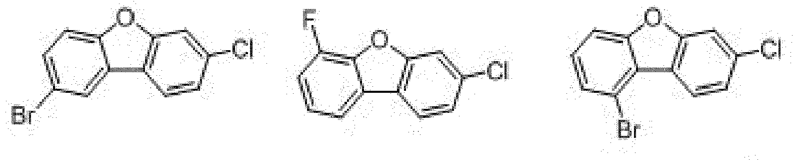

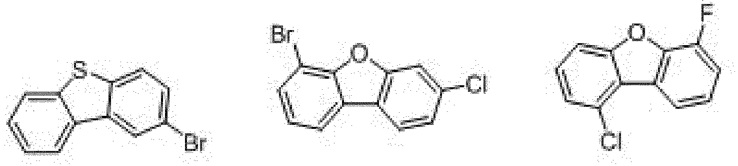

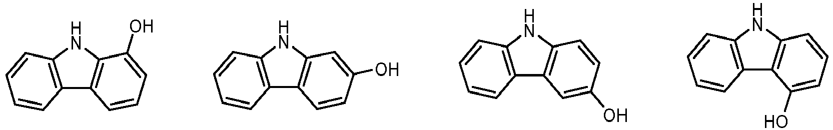

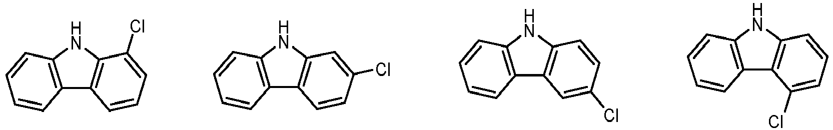

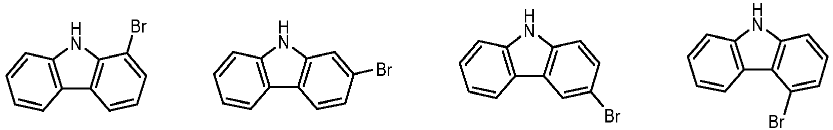

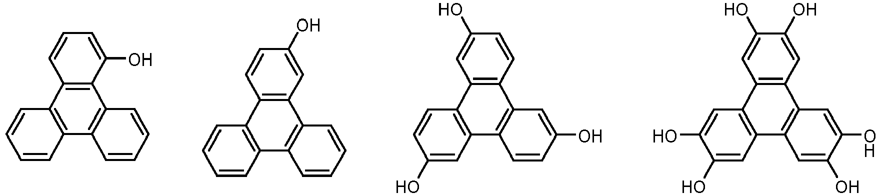

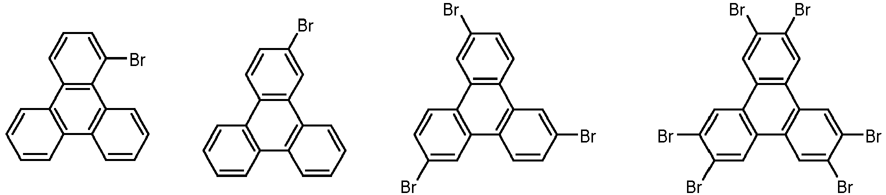

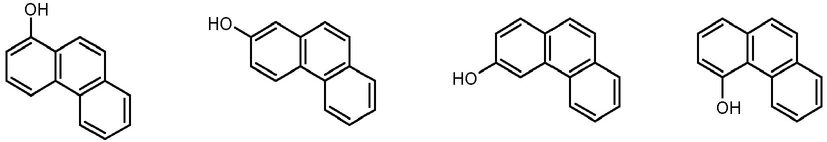

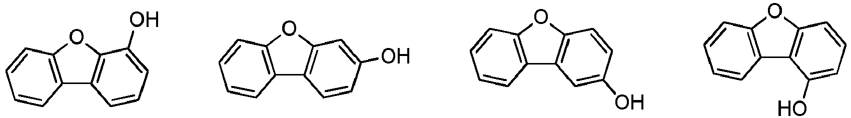

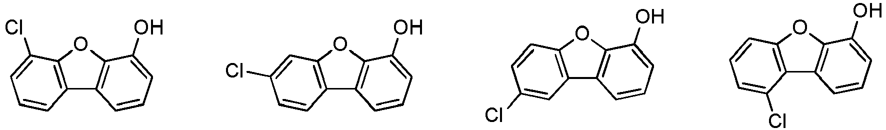

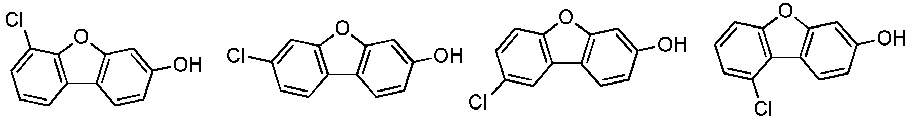

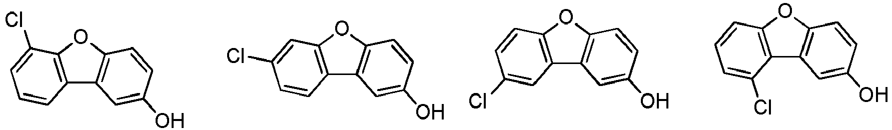

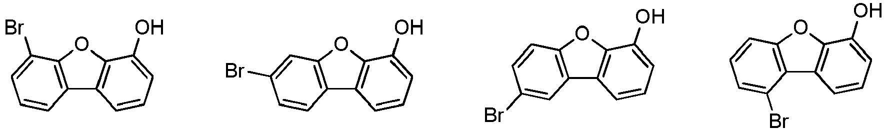

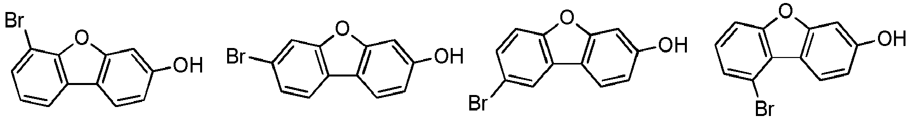

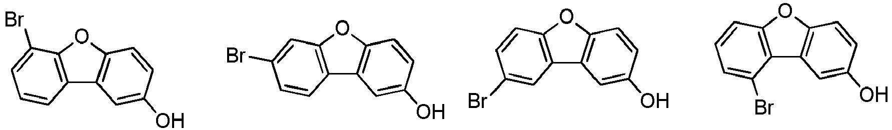

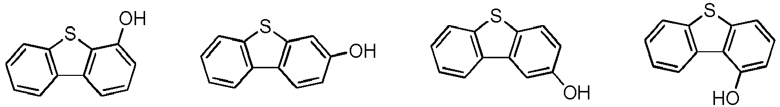

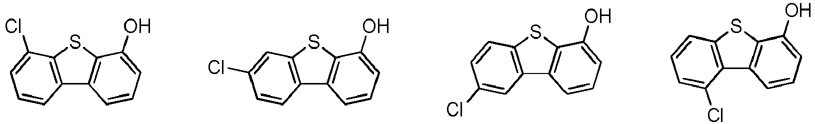

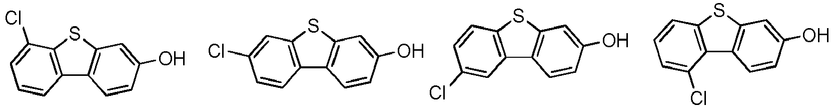

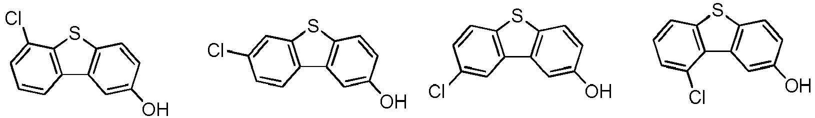

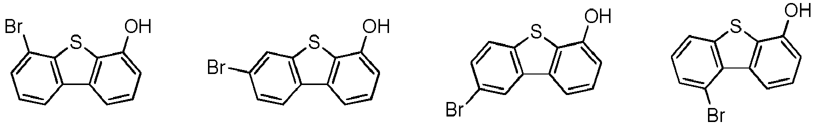

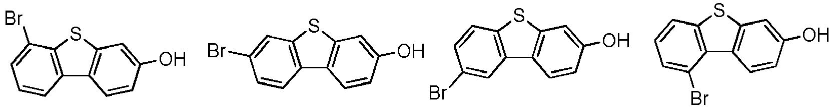

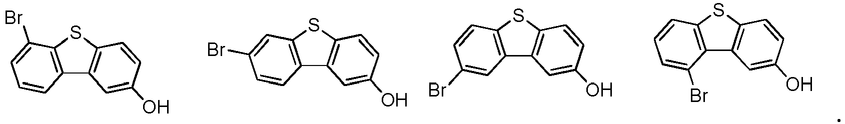

The method for producing a deuterated aromatic compound of the present specification can further include substituting the internal air of the reactor with nitrogen or an inert gas.

In the performing of deuteration of the aromatic compound, deuteration can be performed at room temperature without applying heat, or deuteration can be performed by heating the solution. In this case, the room temperature is a natural temperature at which the compound is not heated or cooled, and can be specifically in a range of 20±5°C.

In the method for producing a deuterated aromatic compound of the present specification, the performing of the deuterated reaction of the aromatic compound can include:
preparing a solution including an aromatic compound including one or more aromatic rings, heavy water (D₂O) and an organic compound which can be hydrolyzed by the heavy water; and
performing the deuterated reaction of the aromatic compound by heating the solution.

The performing of the deuterated reaction of the aromatic compound by heating the reactor can be a step of heating the solution at a temperature of 160°C or less, 150°C or less, 140°C or less, 130°C or less, 120°C or less, 110°C or less, 100°C or less, 90°C or less, or 80°C or more, specifically, a temperature of 80°C or more and 140°C or less.

In this case, the deuterium reaction time is reacted for 3 hours or more after the temperature is completely increased. Specifically, the deuterium reaction time can be reacted for 3 hours or more and 24 hours or less, preferably for 6 hours or more and 18 hours or less, after the temperature in the deuterium reaction is completely increased.

The method for producing a deuterated aromatic compound of the present specification further includes obtaining the deuterated aromatic compound after performing the deuteration. The obtaining method can be performed by a method known in the art, and is not particularly limited.

The higher the deuterium substitution rate of the obtained deuterated aromatic compound, the better the deuterium substitution rate, and specifically, the deuterium substitution rate of the obtained deuterated aromatic compound can be 50% or more, 60% or more, 70% or more, 80% or more, 85% or more, 90% or more, 91% or more, 92% or more, 93% or more, 94% or more, 95% or more, 96% or more, 97% or more, 98% or more, 99% or more or 100%.

The higher the purity of the obtained deuterated aromatic compound, the better the purity, and specifically, the purity of the obtained deuterated aromatic compound can be 90% or more, 91% or more, 92% or more, 93% or more, 94% or more, 95% or more, 96% or more, 97% or more, 98% or more, 99% or more or 100%.

The present specification provides a deuterated aromatic compound produced by the above-described production method.

In an exemplary embodiment of the present specification, the deuterated aromatic compound means an aromatic compound which is substituted with at least one or more deuterium atoms.

In an exemplary embodiment of the present specification, the deuterated aromatic compound includes a substituent selected from the group consisting of a leaving group, a hydroxyl group, a substituted or unsubstituted amine group, and a cyano group.

In the present specification, the compound including the leaving group can be an intermediate of a final compound of organic synthesis, and the leaving group means a reaction group which is left based on the final compound, or is chemically modified by being bonded to other reactants. Thus, for the leaving group, the type of leaving group and the position to which the leaving group is bonded are determined by the method of organic synthesis and the position of the substituent of the final compound.

In an exemplary embodiment of the present specification, the leaving group can be selected from the group consisting of a halogen group and a boronic acid group.

In an exemplary embodiment of the present specification, a deuterated aromatic compound including the substituent selected from the group consisting of a leaving group, a hydroxyl group, a substituted or unsubstituted amine group and a cyano group can be any one compound of the following Chemical Formulae 7 to 11: 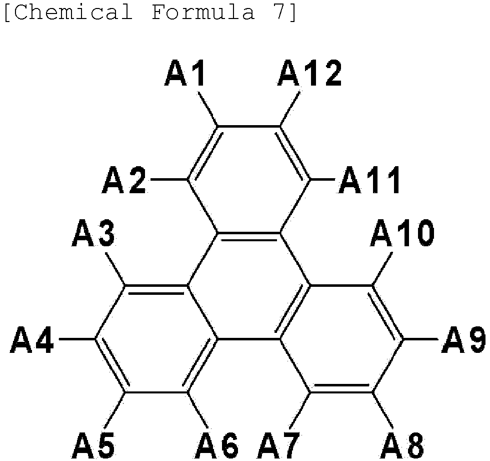 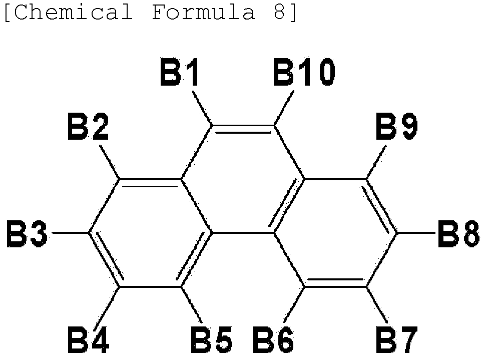 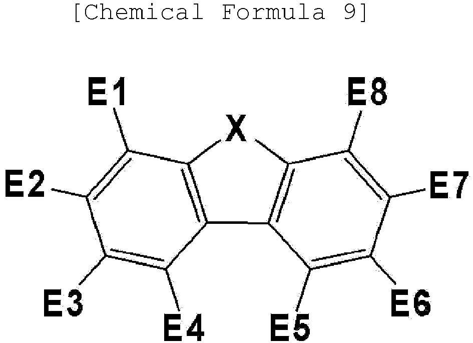 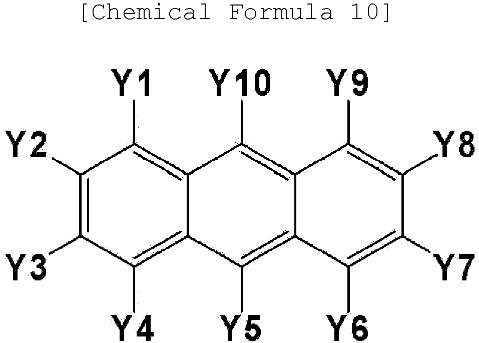 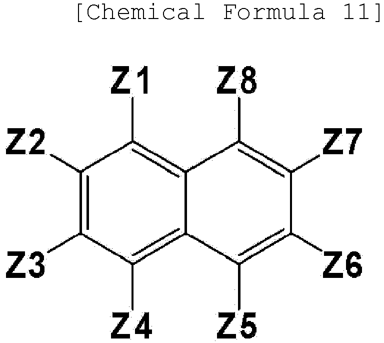 wherein in Chemical Formulae 7 to 11:
X is O, S or NR;
R is hydrogen, deuterium, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group;
at least one of A1 to A12 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
at least one of B1 to B10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
at least one of E1 to E8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring;
at least one of Y1 to Y10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring; and
at least one of Z1 to Z8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group; a substituted or unsubstituted alkyl group, a substituted or unsubstituted cycloalkyl group, a substituted or unsubstituted aryl group, or a substituted or unsubstituted heterocyclic group, or can be bonded to an adjacent group to form a substituted or unsubstituted ring.

In an exemplary embodiment of the present specification, the compounds of Chemical Formulae 7 to 11 each have a substituent selected from the group consisting of a leaving group, a hydroxyl group, a substituted or unsubstituted amine group and a cyano group.

In an exemplary embodiment of the present specification, a deuterated aromatic compound including the substituent selected from the group consisting of a leaving group, a hydroxyl group, a substituted or unsubstituted amine group and a cyano group is any one of the following compounds, and the compounds are each substituted with one or more deuteriums:

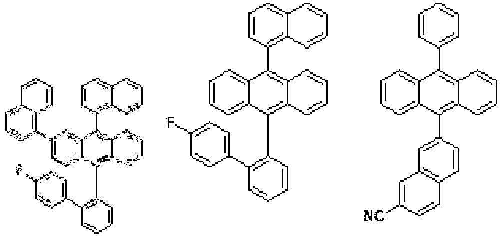

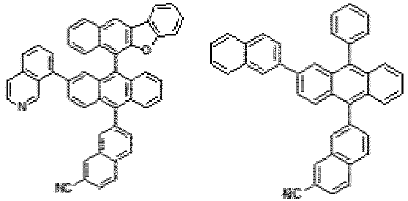

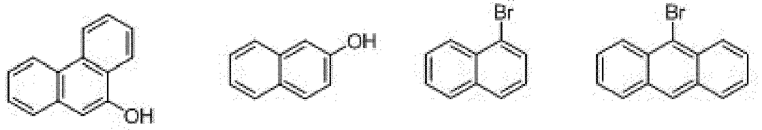

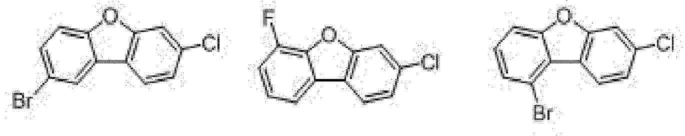

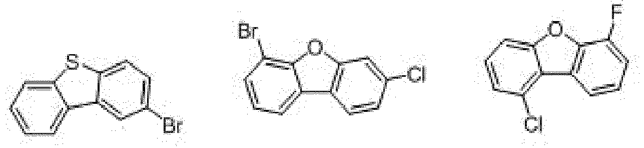

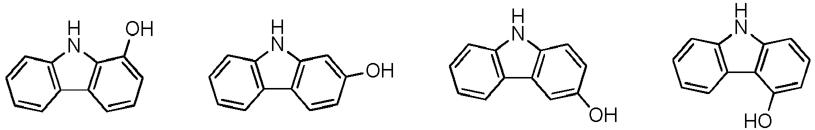

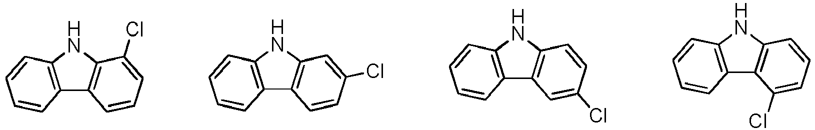

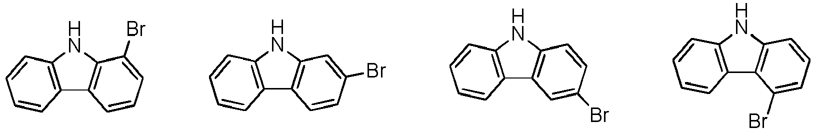

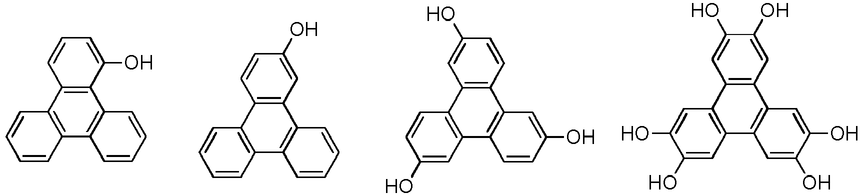

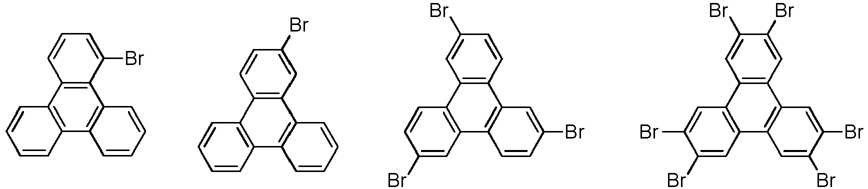

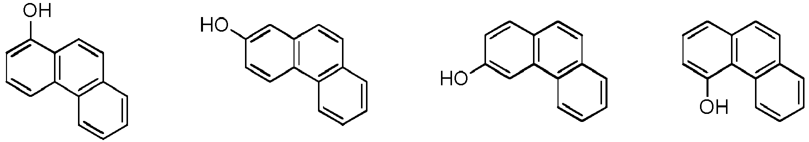

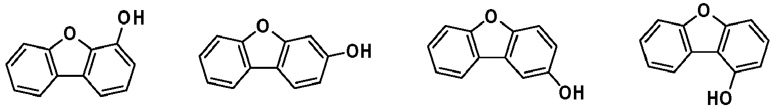

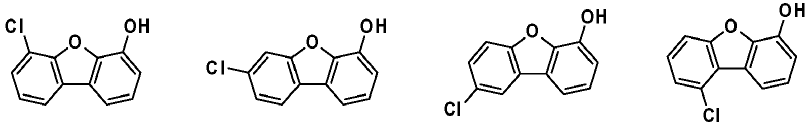

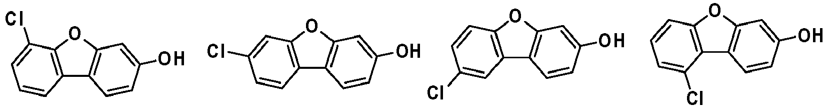

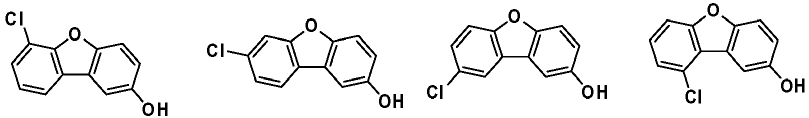

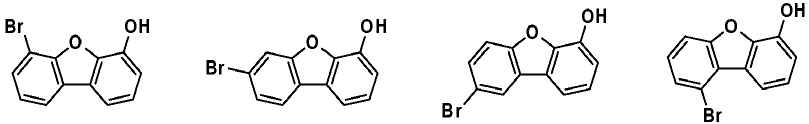

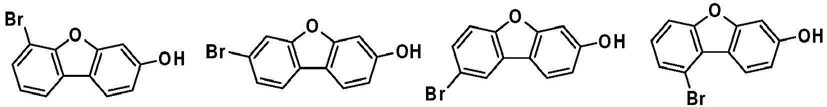

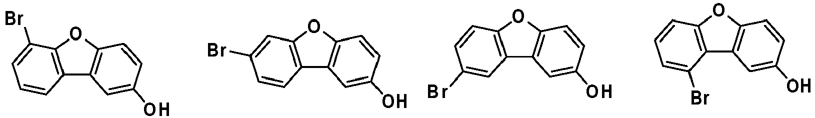

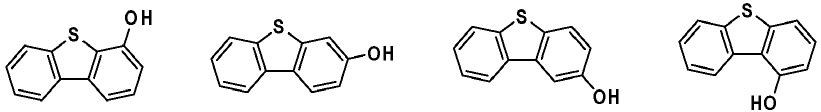

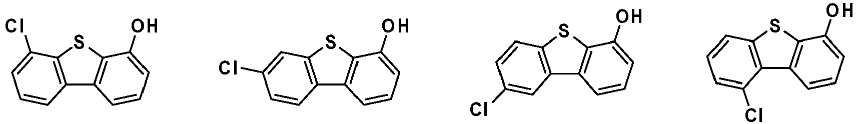

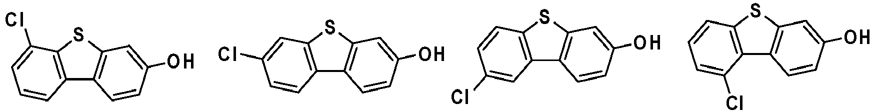

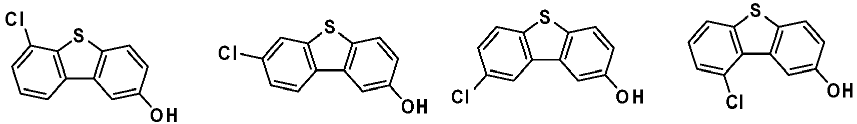

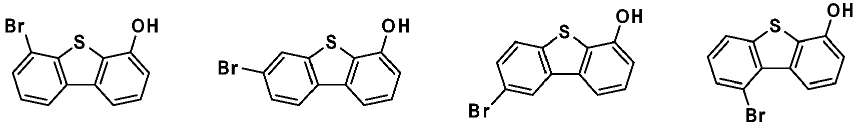

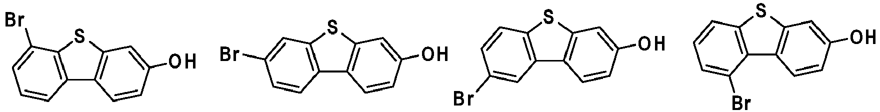

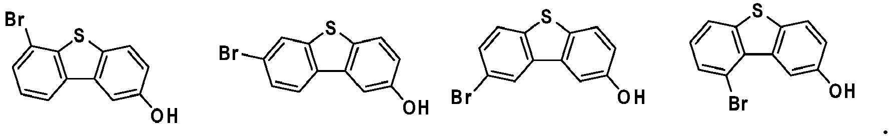

Theoretically, when all of the hydrogens in the deuterated compound are substituted with deuterium, that is, when the deuterium substitution rate is 100%, the service life characteristics are most ideally improved. However, there are problems such as the need for extreme conditions due to steric hindrance and the destruction of the compound before the compound is deuterated due to side reactions, and in reality, it is difficult to replace all of the hydrogen of a compound at a deuterated substitution rate of 100%, and even when a deuterated substitution rate of nearly 100% is obtained, the efficiency compared to investment is not good in consideration of process time, cost, and the like.

In the present specification, since a deuterated compound produced by a deuterated reaction and having one or more deuteriums is produced as a composition having two or more isotopes having different molecular weights depending on the number of substituted deuteriums, the position where deuterium is substituted in the structure will be omitted.

In the compound having the structure, at least one of the positions which are indicated by hydrogen or in which substituted hydrogen is omitted can be substituted with deuterium.

The present specification provides a deuterated reaction composition including an aromatic compound including one or more aromatic rings, heavy water (D₂O) and an organic compound which can be hydrolyzed by the heavy water.

For the deuterated reaction composition, the description on the solution in the above-described production method can be cited.

In an exemplary embodiment of the present specification, the organic compound which can be hydrolyzed by the heavy water can include at least one compound of the following Chemical Formulae 1 to 4:

[Chemical Formula 1] R1-C(O)OC(O)-R2

[Chemical Formula 2] R3-S(O₂)OS(O₂)-R4

[Chemical Formula 3] R5-C(O)O-R6

[Chemical Formula 4] R7-CONH-R8

wherein in Chemical Formulae 1 to 4:
R1 to R8 are the same as or different from each other, and are each independently a monovalent organic group.

In an exemplary embodiment of the present specification, R1 to R8 are the same as or different from each other, and can be each independently an alkyl group which is unsubstituted or substituted with a halogen group, or an aryl group which is unsubstituted or substituted with a halogen group.

In an exemplary embodiment of the present specification, R1 to R8 are the same as or different from each other, and can be each independently a substituent of the following Chemical Formula 5 or 6:

[Chemical Formula 5] - (CH₂)ₗ(CF₂)ₘ(CF₃)ₙ(CH₃)₁₋ₙ

[Chemical Formula 6] -C(H)ₐ((CH₂)ₗ(CF₂)ₘCF₃)₃₋ₐ

wherein in Chemical Formulae 5 and 6:
l and m are each an integer from 0 to 10; and n and a are each 0 or 1.

In an exemplary embodiment of the present specification, the organic compound which can be hydrolyzed by the heavy water can include at least one of trifluoromethanesulfonic anhydride, trifluoroacetic anhydride, acetic anhydride and methanesulfonic anhydride.

The present specification provides an electronic device including the above-described deuterated aromatic compound.

The present specification provides a method for manufacturing an electronic device, the method including: manufacturing an electronic device using the above-described deuterated aromatic compound.

For the electronic device and the method for manufacturing an electronic device, the description of the composition above can be cited, and the repeated description will be omitted.

The electronic device is not particularly limited as long as the electronic device can use the above-described deuterated aromatic compound, and can be, for example, an organic light emitting device, an organic phosphorescent device, an organic solar cell, an organic photo conductor, an organic transistor, or the like.

The electronic device includes: a first electrode; a second electrode provided to face the first electrode; and an organic material layer having one or more layers provided between the first electrode and the second electrode, and one or more layers of the organic material layer can include the above-described deuterated aromatic compound.

The present specification provides an organic light emitting device including the above-described deuterated aromatic compound.

In an exemplary embodiment of the present specification, the organic light emitting device includes: a first electrode; a second electrode provided to face the first electrode; and an organic material layer provided between the first electrode and the second electrode, in which the organic material layer includes the deuterated aromatic compound.

In an exemplary embodiment of the present specification, the organic material layer includes a light emitting layer including the deuterated aromatic compound.

The organic material layer of the organic light emitting device of the present specification can also be composed of a single-layered structure, but can be composed of a multi-layered structure in which two or more organic material layers are stacked. For example, the organic material layer of the present specification can be composed of one to three layers. Further, the organic light emitting device of the present specification can have a structure including a hole injection layer, a hole transport layer, a light emitting layer, an electron transport layer, an electron injection layer, and the like as organic material layers. However, the structure of the organic light emitting device is not limited thereto, and can include a fewer number of organic layers.

When the organic light emitting device includes a plurality of organic material layers, the organic material layers can be formed of the same material or different materials.

For example, the organic light emitting device of the present specification can be manufactured by sequentially stacking a positive electrode, an organic material layer, and a negative electrode on a substrate. In this case, the organic light emitting device can be manufactured by depositing a metal or a metal oxide having conductivity, or an alloy thereof on a substrate to form a positive electrode, forming an organic material layer including a hole injection layer, a hole transport layer, a light emitting layer, and an electron transport layer thereon, and then depositing a material, which can be used as a negative electrode, thereon, by using a physical vapor deposition (PVD) method such as sputtering or e-beam evaporation. In addition to the method described above, an organic light emitting device can be made by sequentially depositing a negative electrode material, an organic material layer, and a positive electrode material on a substrate.

Further, the deuterated aromatic compound produced by the methods described herein can be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In an exemplary embodiment of the present specification, the first electrode is a positive electrode, and the second electrode is a negative electrode.

According to another exemplary embodiment, the first electrode is a negative electrode, and the second electrode is a positive electrode.

In another exemplary embodiment, the organic light emitting device can be a normal type organic light emitting device in which a positive electrode, an organic material layer having one or more layers, and a negative electrode are sequentially stacked on a substrate.

In still another exemplary embodiment, the organic light emitting device can be an inverted type organic light emitting device in which a negative electrode, an organic material layer having one or more layers, and a positive electrode are sequentially stacked on a substrate.

In the present specification, the materials for the negative electrode, the organic material layer and the positive electrode are not particularly limited except for including an aromatic compound deuterated in at least one layer of the organic material layer, and a material known in the art can be used.

In the present specification, the above-described deuterated aromatic compound can be used by a principle which is similar to the principle applied to an organic light emitting device, even in an electronic device including an organic phosphorescent device, an organic solar cell, an organic photo conductor, an organic transistor, and the like. For example, the organic solar cell can have a structure including a negative electrode, a positive electrode, and a photoactive layer provided between the negative electrode and the positive electrode, and the photoactive layer can include the selected deuterated compound.

### Examples

Hereinafter, the present specification will be described in more detail through Examples. However, the following Examples are provided only for exemplifying the present specification, but are not intended to limit the present specification.

### [Examples]

### [Example 1]

First, 1 g of naphthalene, 58 g of methanesulfonic anhydride and 11 ml of heavy water were introduced into a flask. Then, a reaction was performed at a temperature of 80°C for 18 hours by heating the flask while stirring the mixture. After the reaction was completed, the flask was cooled using ice water and 20 ml of ethyl acetate was added thereto. Then, salts were produced by adding 50 ml of pyridine thereto. The resulting salts were removed using a filter, and the filtrate was neutralized by introducing 20 ml of an aqueous 10% potassium carbonate (K₂CO₃) solution. Here, after only the organic solvent layer was isolated and the residual moisture was removed using magnesium sulfate (MgSO₄), the residue was filtered and then naphthalene substituted with deuterium was obtained by removing the solvent using a rotary evaporator.

### [Example 2]

Anthracene substituted with deuterium was obtained by adding anthracene instead of napthalene thereto to perform the deuterium substitution reaction in the same manner as in Example 1.

### [Example 3]

9-phenylanthracene substituted with deuterium was obtained by adding 9-phenylanthracene instead of naphthalene thereto to perform the deuterium substitution reaction in the same manner as in Example 1.

### [Example 4]

9-bromoanthracene substituted with deuterium was obtained by adding 9-bromoanthracene instead of naphthalene thereto to perform the deuterium substitution reaction in the same manner as in Example 1.

### [Example 5]

9-anthraceneboronic acid substituted with deuterium was obtained by adding 9-anthraceneboronic acid instead of naphthalene thereto to perform the deuterium substitution reaction in the same manner as in Example 1.

### [Example 6]

Naphthalene substituted with deuterium was obtained by adding 54 ml of trifluoromethanesulfonic anhydride instead of methanesulfonic anhydride thereto to perform the deuterium substitution reaction in the same manner as in Example 1.

### [Example 7]

Naphthalene substituted with deuterium was obtained by adding 20 g of methanesulfonic anhydride and 40 ml of acetic anhydride instead of 58 g of methanesulfonic anhydride thereto to perform the deuterium substitution reaction in the same manner as in Example 1.

### [Example 8]

Naphthalene substituted with deuterium was obtained by adding 20 g of methanesulfonic anhydride and 40 ml of methyl acetate instead of 58 g of methanesulfonic anhydride thereto to perform the deuterium substitution reaction in the same manner as in Example 1.

### [Example 9]

Naphthalene substituted with deuterium was obtained by adding 20 ml of trifluoromethanesulfonic anhydride and 40 ml of ethyl acetate instead of 58 g of methanesulfonic anhydride thereto to perform the deuterium substitution reaction in the same manner as in Example 1.

### [Example 10]

Naphthalene substituted with deuterium was obtained by adding 20 g of methanesulfonic anhydride and 40 ml of dimethylacetamide instead of 58 g of methanesulfonic anhydride thereto to perform the deuterium substitution reaction in the same manner as in Example 1.

### [Example 11]

Naphthalene substituted with deuterium was obtained by adding 20 ml of trifluoromethanesulfonic anhydride and 40 ml of dimethylacetamide instead of 58 g of methanesulfonic anhydride thereto to perform the deuterium substitution reaction in the same manner as in Example 1.

### [Example 12]

9-bromoanthracene substituted with deuterium was obtained by adding 20 ml of trifluoromethanesulfonic anhydride and 40 ml of dimethylacetamide instead of 58 g of methanesulfonic anhydride thereto to perform the deuterium substitution reaction in the same manner as in Example 4.

### [Example 13]

9H-carbazole substituted with deuterium was obtained by adding 9H-carbazole instead of naphthalene thereto to perform the deuterium substitution reaction in the same manner as in Example 1.

### [Example 14]

3-bromo-9H-carbazole substituted with deuterium was obtained by adding 3-bromo-9H-carbazole instead of naphthalene thereto to perform the deuterium substitution reaction in the same manner as in Example 1.

### [Example 15]

9-chlorodibenzo[b,d]furan-2-ol substituted with deuterium was obtained by adding 9-chlorodibenzo[b,d]-furan-2-ol instead of naphthalene thereto to perform the deuterium substitution reaction in the same manner as in Example 1.

### [Example 16]

Dibenzo[b,d]thiophene substituted with deuterium was obtained by adding dibenzo[b,d]thiophene instead of naphthalene thereto to perform the deuterium substitution reaction in the same manner as in Example 1.

### [Example 17]

5,11-dihydroindolo[3,2-b]carbazole substituted with deuterium was obtained by adding 5,11-dihydroindolo[3,2-b]carbazole instead of naphthalene thereto to perform the deuterium substitution reaction in the same manner as in Example 6.

### [Example 18]

8-bromo-1-chlorodibenzo[b,d]furan substituted with deuterium was obtained by adding 8-bromo-1-chlorodibenzo[b,d]furan instead of naphthalene thereto to perform the deuterium substitution reaction in the same manner as in Example 6.

### [Comparative Example 1]

1 g of naphthalene, 15 ml of heavy water (D₂O), and 0.5 g of Pt/C (the content of Pt in the catalyst was 10 wt%) were put into a high-pressure reactor and the inside of the reactor was sealed by covering the head of the reactor. Hydrogen was blown into the reactant for about 5 minutes with stirring. Then, the reaction was performed under the hydrogen atmosphere at a temperature of 180°C for 18 hours. After the deuterium substitution reaction was completed, the temperature was lowered, 15 ml of methylene chloride was added thereto, the resulting mixture was stirred and filtered, and the catalyst was removed. Then, after only the organic solvent layer was isolated and the residual moisture was removed using MgSO₄, the residue was filtered and then naphthalene substituted with deuterium was obtained by removing the solvent using a rotary evaporator.

### [Comparative Example 2]

Anthracene substituted with deuterium was obtained by performing the deuterium substitution reaction on anthracene instead of naphthalene in the same manner as in Comparative Example 1.

### [Comparative Example 3]

The deuterium substitution reaction was performed by adding 9-bromoanthracene instead of naphthalene thereto in the same manner as in Comparative Example 1. As a result, 9-bromoanthracene substituted with deuterium was obtained, but anthracene substituted with deuterium, which lost most of the bromine group could be confirmed.

### [Comparative Example 4]

9H-carbazole substituted with deuterium was obtained by performing the deuterium substitution reaction on 9H-carbazole instead of naphthalene in the same manner as in Comparative Example 1.

### [Comparative Example 5]

3-bromo-9H-carbazole substituted with deuterium was obtained by performing the deuterium substitution reaction on 3-bromo-9H-carbazole instead of naphthalene in the same manner as in Comparative Example 1.

### [Comparative Example 6]

8-bromo-1-chlorodibenzo[b,d]furan substituted with deuterium was obtained by performing the deuterium substitution reaction on 8-bromo-1-chlorodibenzo[b,d]-furan instead of naphthalene in the same manner as in Comparative Example 1.

### [Experimental Examples]

The purity, deuterium substitution rate, and hydrogenated compound proportion for Examples 1 to 12 and Comparative Examples 1 to 3 were measured, and the results are shown in the following Table 1.

The purity and hydrogenated compound proportion were obtained by dissolving the completely reacted specimen in a tetrahydrofuran solvent for high performance liquid chromatography (HPLC) to integrate the spectrum at a wavelength of 254 nm through HPLC. In this case, as a mobile phase solvent, a solvent in which acetonitrile and tetrahydrofuran were mixed at a ratio of 5:5 and 1% formic acid was mixed and water were used.

A sample specimen obtained by quantifying a specimen completely subjected to deuterated reaction and dissolving the specimen in a solvent for NMR measurement, and an internal standard specimen obtained by quantifying any compound whose peak does not overlap with the compound before the deuterated reaction in the same amount as the above specimen and dissolving the compound in the same solvent for NMR measurement were prepared. NMR measurement graphs were obtained each using ¹H-NMR for the prepared sample specimen and internal standard specimen.

When the ¹H-NMR peak was assigned, a relative integration value for each position of the specimen completely subjected to deuterated reaction was obtained by setting the internal standard peak to 1.

When the specimen completely subjected to deuterated reaction is substituted with deuterium at all positions, no peak related to hydrogen appears, and in this case, the deuterium substitution rate is determined to be 100%. In contrast, when hydrogen at all positions is not substituted with deuterium, a peak of hydrogen that has not been substituted with deuterium will appear.

Based on this result, in the present experiment, a deuterium substitution rate is obtained by subtracting an integration value of a peak due to unsubstituted hydrogen in the NMR measurement graph of the sample specimen from an integration value of a peak related to hydrogen in the NMR measurement graph of the internal standard specimen in which deuterium is not substituted. This value is an integration value relative to each position, does not appear as the corresponding peak due to substitution with deuterium, and indicates a ratio of substitution with deuterium.

And then, a substitution rate for each position of the specimen was calculated using the weight of the specimen used when the ¹H-NMR measurement sample is prepared, the weight of the internal standard, and the relative integration value.

**[Table 1]**

| | Reactant | Hydrolyzable compound | Purity (%) | Deuterium substitution rate (%) | Hydrogenated compound proportion (%) | Reaction temperature (°C) | Reaction pressure (bar) |
|---|---|---|---|---|---|---|---|
| Example 1 | Naphthalene | Methanesulfonic anhydride | 98.2 | 94 | 0 | 80 | Normal pressure |
| Example 2 | Anthracene | Methanesulfonic anhydride | 97.1 | 95 | 0 | 80 | Normal pressure |
| Example 3 | 9-phenylanthracene | Methanesulfonic anhydride | 97.2 | 91 | 0 | 80 | Normal pressure |
| Example 4 | 9-bromoanthrancene | Methanesulfonic anhydride | 94.9 | 91 | 0 | 80 | Normal pressure |
| Example 5 | 9-Anthraceneboronic acid | Methanesulfonic anhydride | 96.1 | 92 | 0 | 80 | Normal pressure |
| Example 6 | Naphthalene | Trifluoromethanesulfonic anhydride | 97.4 | 89 | 0 | 80 | Normal pressure |
| Example 7 | Naphthalene | Methanesulfonic anhydride and acetic anhydride | 96.3 | 89 | 0 | 80 | Normal pressure |
| Example 8 | Naphthalene | Methanesulfonic anhydride and methyl acetate | 97.8 | 91 | 0 | 80 | Normal pressure |
| Example 9 | Naphthalene | Trifluoromethanesulfonic anhydride and ethyl acetate | 95.4 | 95 | 0 | 80 | Normal pressure |
| Example 10 | Naphthalene | Methanesulfonic anhydride and dimethylacetamide | 95.8 | 94 | 0 | 80 | Normal pressure |
| Example 11 | Naphthalene | Trifluoromethanesulfonic anhydride and dimethylacetamide | 95.2 | 92 | 0 | 80 | Normal pressure |
| Example 12 | 9-bromoanthrancene | Trifluoromethanesulfonic anhydride and dimethylacetamide | 94.4 | 90 | 0 | 80 | Normal pressure |
| Example 13 | 9H-carbazole | Methanesulfonic anhydride | 99.1 | 97 | 0 | 80 | Normal pressure |
| Example 14 | 3-bromo-9H-carbazole | Methanesulfonic anhydride | 99.4 | 95 | 0 | 80 | Normal pressure |
| Example 15 | 9-chlorodibenzo [b,d]furan -2-ol | Methanesulfonic anhydride | 98.9 | 95 | 0 | 80 | Normal pressure |
| Example 16 | Dibenzo[b,d]thiophene | Methanesulfonic anhydride | 99.6 | 97 | 0 | 80 | Normal pressure |
| Example 17 | 5,11-dihydroindolo[3,2-b]carbazole | Trifluoromethanesulfonic anhydride | 99.4 | 96 | 0 | 80 | Normal pressure |
| Example 18 | 8-bromo-1-chlorodibenzo [b,d]furan | Trifluoromethanesulfonic anhydride | 99.3 | 97 | 0 | 80 | Normal pressure |
| Comparative Example 1 | Naphthalene | - | 85.1 | 94 | 10 | 180 | 9.7 |
| Comparative Example 2 | Anthracene | - | 87.1 | 95 | 8 | 180 | 10.2 |
| Comparative Example 3 | 9-bromoanthrancene | - | 42.3 | 80 | 20 | 180 | 9.9 |
| Comparative Example 4 | 9H-carbazole | - | 86.2 | 97 | 4.5 | 180 | 9.7 |
| Comparative Example 5 | 3-bromo-9H-carbazole | - | 47.7 | 61 | 6.7 | 180 | 10.2 |
| Comparative Example 6 | 8-bromo-1-chlorodibenzo[b,d]furan | - | 31.5 | 57 | 3.2 | 180 | 9.9 |

Through the results in Table 1, it can be confirmed that a high-purity deuterium compound can be obtained while the deuterium substitution rate is at the equivalent level under relatively low temperature and pressure conditions when the examples are compared with the comparative examples. This is because the production of hydride compounds, which is a side reaction, was reduced and the halogen element was still maintained until the deuterated reaction was completed.

## Claims

1. A method for producing a deuterated aromatic compound, the method comprising: performing a deuterated reaction of an aromatic compound comprising one or more aromatic rings using a solution comprising the aromatic compound, heavy water, and an organic compound which can be hydrolyzed by the heavy water.

2. The method of claim 1, wherein the organic compound which can be hydrolyzed by the heavy water comprises at least one compound of the following Chemical Formulae 1 to 4:
[Chemical Formula 1] R1-C(O)OC(O)-R2
[Chemical Formula 2] R3-S(O₂)OS(O₂)-R4
[Chemical Formula 3] R5-C(O)O-R6
[Chemical Formula 4] R7-CONH-R8
wherein in Chemical Formulae 1 to 4:
R1 to R8 are the same as or different from each other, and are each independently a monovalent organic group.

3. The method of claim 2, wherein R1 to R8 are the same as or different from each other, and are each independently an alkyl group which is unsubstituted or substituted with a halogen group, or an aryl group which is unsubstituted or substituted with a halogen group.

4. The method of claim 2, wherein R1 to R8 are the same as or different from each other, and are each independently a substituent of the following Chemical Formula 5 or 6:
[Chemical Formula 5] - (CH₂)ₗ(CF₂)ₘ(CF₃)ₙ(CH₃)₁₋ₙ
[Chemical Formula 6] -C (H)ₐ((CH₂)ₗ(CF₂)ₘCF₃)₃₋ₐ
wherein in Chemical Formulae 5 and 6:
l and m are each an integer from 0 to 10; and
n and a are each 0 or 1.

5. The method of claim 2, wherein the organic compound which can be hydrolyzed by the heavy water comprises at least one of the compound of Chemical Formula 3 and the compound of Chemical Formula 4, and further comprises at least one of the compound of Chemical Formula 1 and the compound of Chemical Formula 2.

6. The method of claim 1, wherein the organic compound which can be hydrolyzed by the heavy water comprises at least one of trifluoromethanesulfonic anhydride, trifluoroacetic anhydride, acetic anhydride and methanesulfonic anhydride.

7. The method of claim 1, wherein the solution does not comprise a metal catalyst.

8. The method of claim 1, wherein the performing of the deuterated reaction of the aromatic compound comprises:
preparing the solution comprising the aromatic compound comprising one or more aromatic rings, heavy water, and the organic compound which can be hydrolyzed by the heavy water; and
performing the deuterated reaction of the aromatic compound by heating the solution.

9. A deuterated reaction composition, comprising an aromatic compound comprising one or more aromatic rings, heavy water and an organic compound which can be hydrolyzed by the heavy water.

10. The deuterated reaction composition of claim 9, wherein the organic compound which can be hydrolyzed by the heavy water comprises at least one compound of the following Chemical Formulae 1 to 4:
[Chemical Formula 1] R1-C(O)OC(O)-R2
[Chemical Formula 2] R3-S(O₂)OS(O₂)-R4
[Chemical Formula 3] R5-C(O)O-R6
[Chemical Formula 4] R7-CONH-R8
wherein in Chemical Formulae 1 to 4:
R1 to R8 are the same as or different from each other, and are each independently a monovalent organic group.

11. The deuterated reaction composition of claim 10, wherein R1 to R8 are the same as or different from each other, and are each independently an alkyl group which is unsubstituted or substituted with a halogen group, or an aryl group which is unsubstituted or substituted with a halogen group.

12. The deuterated reaction composition of claim 10, wherein R1 to R8 are the same as or different from each other, and are each independently a substituent of the following Chemical Formula 5 or 6:
[Chemical Formula 5] - (CH₂)ₗ(CF₂)ₘ(CF₃)ₙ(CH₃)₁₋ₙ
[Chemical Formula 6] -C (H)ₐ((CH₂)ₗ(CF₂)ₘCF₃)₃₋ₐ
wherein in Chemical Formulae 5 and 6:
l and m are each an integer from 0 to 10; and
n and a are each 0 or 1.

13. The deuterated reaction composition of claim 9, wherein the organic compound which can be hydrolyzed by the heavy water comprises at least one of trifluoromethanesulfonic anhydride, trifluoroacetic anhydride, acetic anhydride and methanesulfonic anhydride.

14. A deuterated aromatic compound produced by the method of any one of claims 1 to 8.

15. The deuterated aromatic compound of claim 14, wherein the deuterated aromatic compound comprises a substituent selected from the group consisting of a leaving group, a hydroxyl group, a substituted or unsubstituted amine group, and a cyano group.

16. The deuterated aromatic compound of claim 15, wherein the leaving group is selected from the group consisting of a halogen group and a boronic acid group.

17. The deuterated aromatic compound of claim 15, wherein the deuterated aromatic compound comprising the substituent selected from the group consisting of a leaving group, a hydroxyl group, a substituted or unsubstituted amine group, and a cyano group is any one of the following Chemical Formulae 7 to 11: 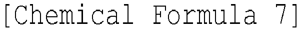 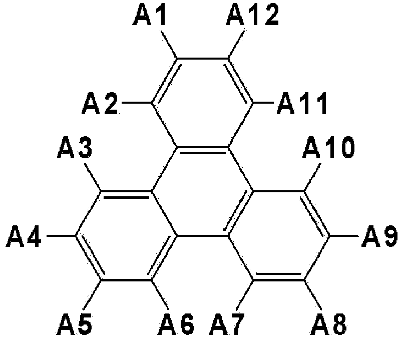 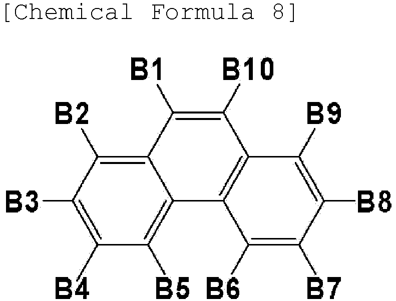 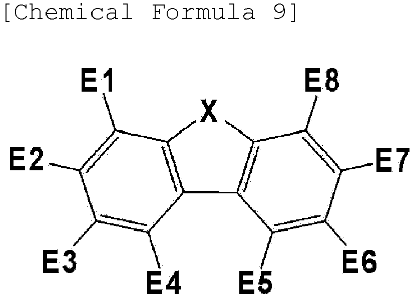 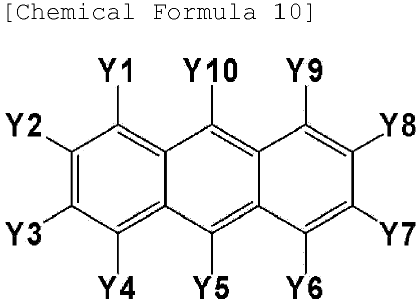 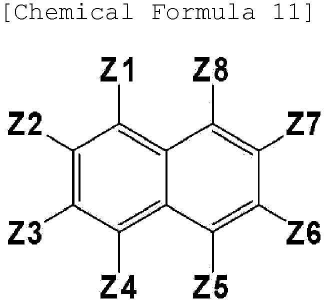 wherein in Chemical Formulae 7 to 11:
X is O, S or NR;
R is hydrogen, deuterium, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group;
at least one of A1 to A12 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group; a substituted or unsubstituted aryl group, or a substituted or unsubstituted heterocyclic group, or are optionally bonded to an adjacent group to form a substituted or unsubstituted ring;
at least one of B1 to B10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring;
at least one of E1 to E8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring;
at least one of Y1 to Y10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring; and
at least one of Z1 to Z8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring.

18. The deuterated aromatic compound of claim 15, wherein the deuterated aromatic compound comprising the substituent selected from the group consisting of a leaving group, a hydroxyl group, a substituted or unsubstituted amine group and a cyano group is any one of the following compounds, and the compounds are each substituted with one or more deuteriums:

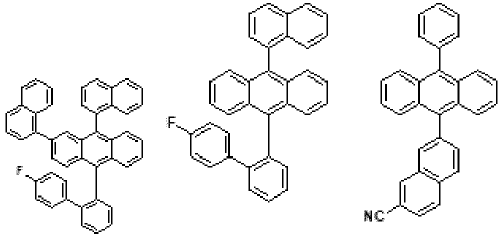

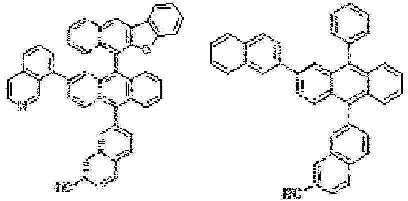

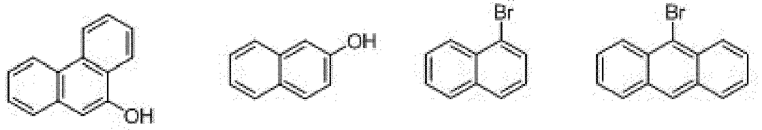

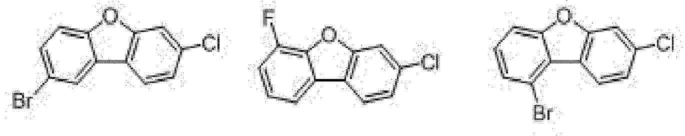

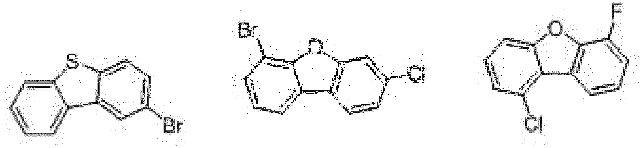

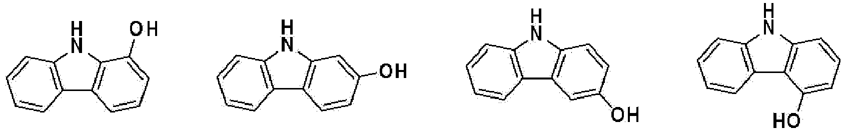

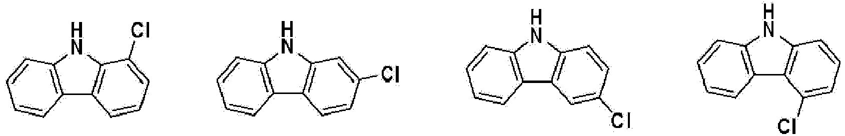

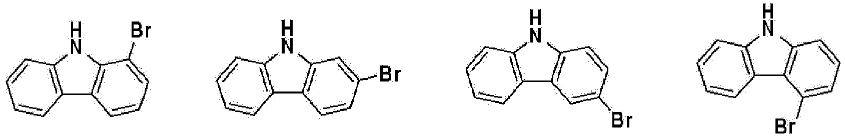

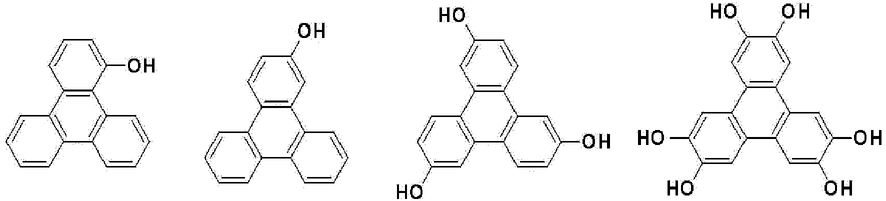

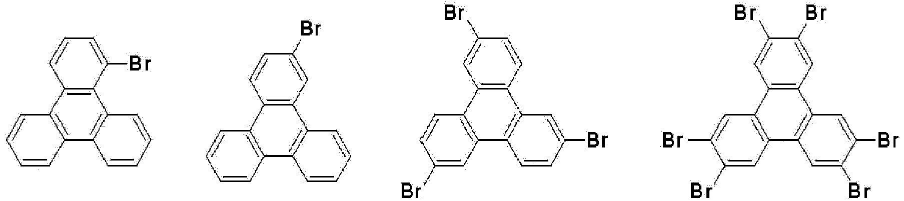

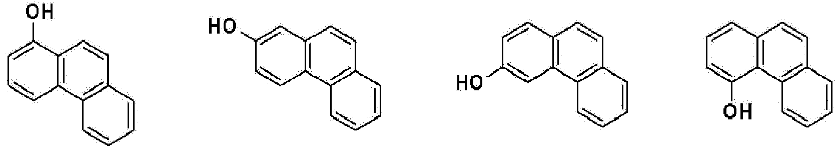

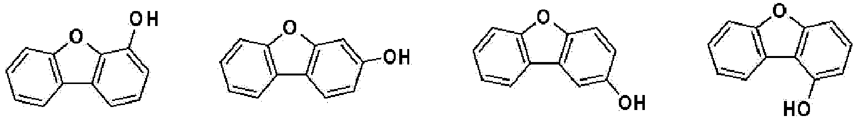

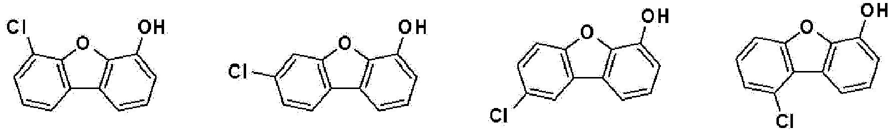

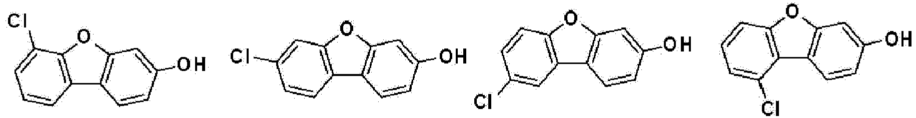

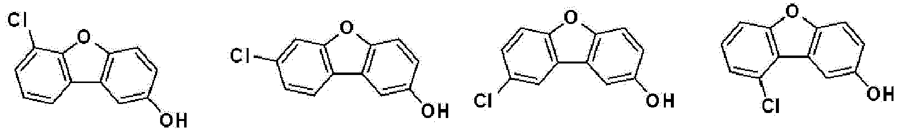

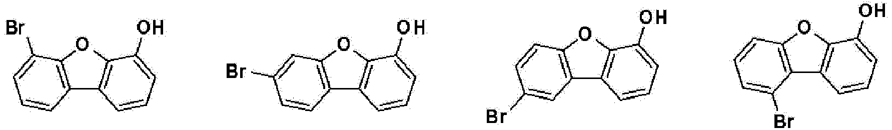

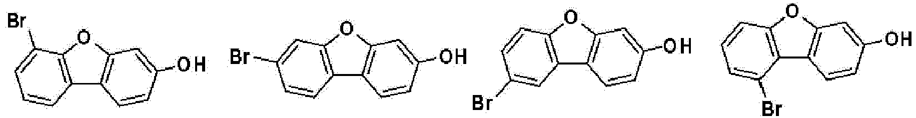

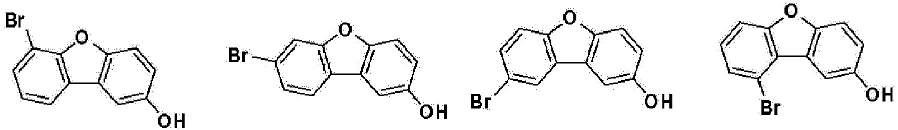

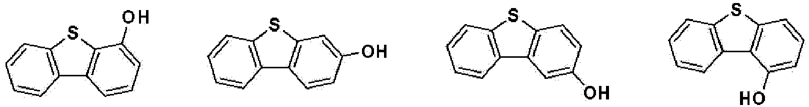

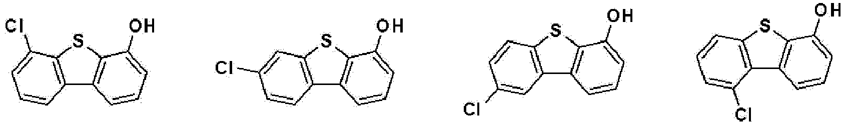

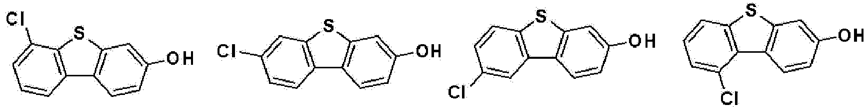

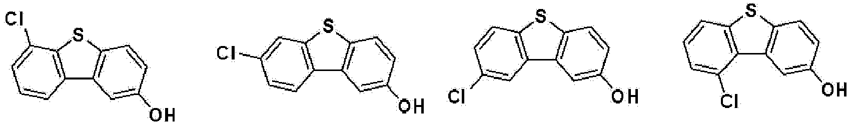

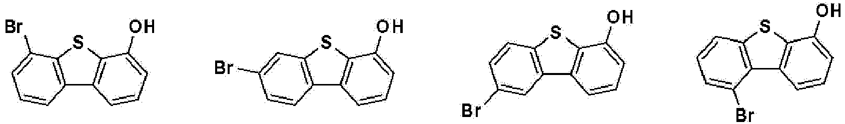

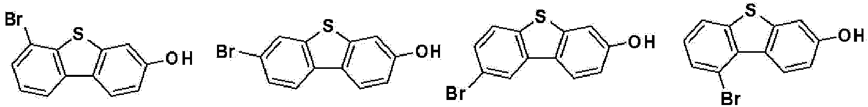

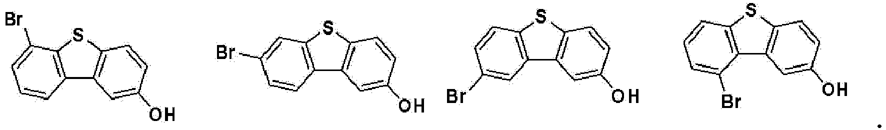

19. A deuterated aromatic compound which is any one of Chemical Formulae 7 to 11: 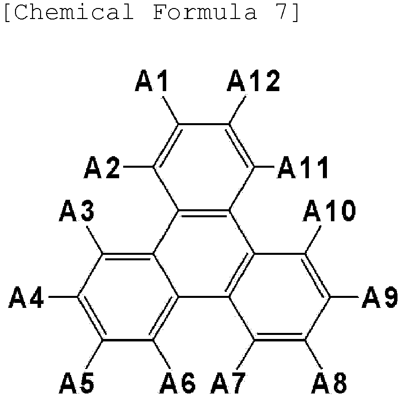 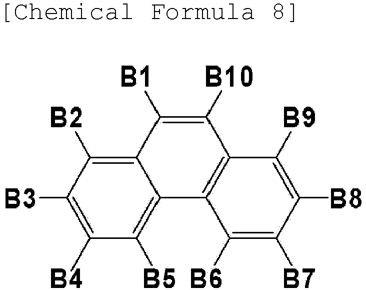 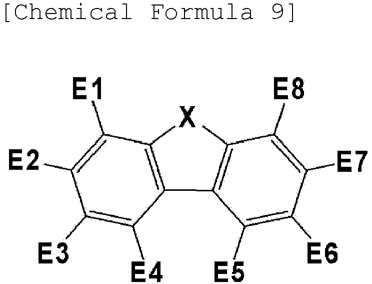 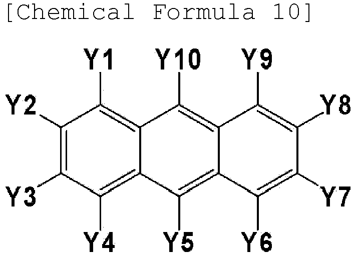 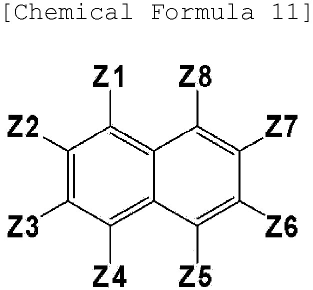 wherein in Chemical Formulae 7 to 11:
X is O, S or NR;
R is hydrogen, deuterium, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group;
at least one of A1 to A12 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring;
at least one of B1 to B10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring;
at least one of E1 to E8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring;
at least one of Y1 to Y10 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring; and
at least one of Z1 to Z8 is deuterium, at least one is a substituent selected from the group consisting of a leaving group, a hydroxyl group, a substituted or unsubstituted amine group and a cyano group, and the others are each independently hydrogen, a leaving group, a hydroxyl group, a substituted or unsubstituted amine group, a cyano group, a substituted or unsubstituted alkyl group, a substituted or unsubstituted cycloalkyl group, a substituted or unsubstituted aryl group, or a substituted or unsubstituted heterocyclic group, or are optionally bonded to an adjacent group to form a substituted or unsubstituted ring.

20. An electronic device comprising the deuterated aromatic compound of claim 14.
